# EUROPEAN PATENT APPLICATION

(11) **EP 4 035 764 A1**
(43) Date of publication of application: **03.08.2022**
(21) Application number: 20880469.0
(22) Date of filing: 30.10.2020
(51) Int. Cl.: B01F 1/00, B01F 3/04, B01F 11/02, A61L 2/20, B08B 9/032, C02F 1/50, C02F 1/58, C02F 1/62, C02F 1/74

(54) **METHOD FOR PRODUCING ULTRA-FINE BUBBLE-CONTAINING LIQUID, ULTRA-FINE BUBBLE-CONTAINING LIQUID, METHOD FOR UTILIZING ULTRA-FINE BUBBLES, AND DEVICE FOR UTILIZING ULTRA-FINE BUBBLES**

(30) Priority: 31.10.2019 JP 2019199138; 26.10.2020 JP 2020178854
(71) Applicant: Canon Kabushiki Kaisha, Ohta-ku Tokyo 146-8501 (JP)
(72) Inventor: KUBOTA Masahiko, Tokyo 146-8501 (JP); YAMAMOTO Akira, Tokyo 146-8501 (JP); YAMADA Akitoshi, Tokyo 146-8501 (JP); IMANAKA Yoshiyuki, Tokyo 146-8501 (JP); YANAI Yumi, Tokyo 146-8501 (JP); ISHINAGA Hiroyuki, Tokyo 146-8501 (JP); OZAKI Teruo, Tokyo 146-8501 (JP); KASHINO Toshio, Tokyo 146-8501 (JP)
(74) Representative: WESER & Kollegen Patentanwälte PartmbB
(86) International application number: PCT/JP2020/040939
(87) International publication number: WO 2021/085629

(57) **Abstract**

Provided are a method for producing an ultra-fine bubble-containing liquid, an ultra-fine bubble-containing liquid, and a method for utilizing and a device for utilizing ultra-fine bubbles that allow highly concentrated UFBs to be maintained for a long period of time and that are capable of effectively utilizing the UFBs. To this end, the method for producing an ultra-fine bubble-containing liquid includes an ultra-fine bubble generating step and a dispersing step to disperse the ultra-fine bubbles. In the ultra-fine bubble generating step, the ultra-fine bubbles are generated in a liquid by heating a heating element and making film boiling on an interface between the liquid and the heating element. In the dispersing step, a floc, which includes two or more ultra-fine bubbles 11, is dispersed into multiple ultra-fine bubbles by applying vibration to the liquid in which the floc floats.

## Description

### Technical Field

The present invention relates to a method for producing an ultra-fine bubble-containing liquid, an ultra-fine bubble-containing liquid, a method for utilizing ultra-fine bubbles, and a device for utilizing ultra-fine bubbles.

### Background Art

Recently, there have been developed techniques for applying the features of fine bubbles such as microbubbles in micrometer-size in diameter and nanobubbles in nanometer-size in diameter. Especially, the utility of ultra-fine bubbles (Ultra Fine Bubble; hereinafter also referred to as "UFBs") smaller than 1.0 µm in diameter has been confirmed in various fields.

In PTL 1, a fine air bubble generating apparatus that generates fine bubbles by jetting a pressurized liquid in which a gas is pressurized and dissolved from a depressurizing nozzle is disclosed. Additionally, in PTL 2, an apparatus that generates fine bubbles by repeating separating and converging of a flow of a gas mixed liquid by using a mixing unit.

### Citation List

### Patent Literature

PTL 1: Japanese Patent No. 6118544
PTL 2: Japanese Patent No. 4456176

### Summary of Invention

### Technical Problem

In any of the apparatuses described in PTLs 1 and 2, in addition to the UFBs of nanometer-size in diameter, relatively a large number of milli-bubbles of millimeter-size in diameter and microbubbles of micrometer-size in diameter are generated. However, because the milli-bubbles and the microbubbles are affected by the buoyancy, the bubbles tend to gradually rise to the liquid surface and disappear during long-time storage.

On the other hand, the UFBs of nanometer-size in diameter are suitable for long-time storage since they are less likely to be affected by the buoyancy and they float in the liquid with Brownian motion. However, if the UFBs are generated with the milli-bubbles and the microbubbles or the gas-liquid interface energy is small, the UFBs are affected by the disappearance of the milli-bubbles and the microbubbles and are decreased over time. That is, even though many UFBs exist during the generation, the number may be decreased at the moment of utilizing the UFBs in practice, and sufficient utilizing effects may not be obtained.

The present invention is made in view of solving the above-described problem. Therefore, an object thereof is to provide a method for producing an ultra-fine bubble-containing liquid, an ultra-fine bubble-containing liquid, and a method for utilizing and a device for utilizing ultra-fine bubbles that allow highly concentrated UFBs to be maintained for a long period of time and that are capable of effectively utilizing the UFBs. Solution to Problem

To this end, a method for producing an ultra-fine bubble-containing liquid of the present invention includes: an ultra-fine bubble generating step to generate an ultra-fine bubble in a liquid by heating a heating element and making film boiling on an interface between the liquid and the heating element; and a dispersing step to disperse a floc, which includes two or more ultra-fine bubbles, into a plurality of ultra-fine bubbles by applying vibration to the liquid in which the floc floats.

Further characteristics of the present invention are disclosed from the following descriptions of embodiments made with reference to the accompanying drawings.

### Advantageous Effects of Invention

According to the present invention, it is possible to maintain highly concentrated UFBs for a long period of time and to effectively utilize the UFBs.

### Brief Description of Drawings

[Fig. 1] Fig. 1 is a diagram illustrating an example of a UFB generating apparatus.
[Fig. 2] Fig. 2 is a schematic configuration diagram of a pre-processing unit.
[Fig. 3] Fig. 3 is a schematic configuration diagram of a dissolving unit and a diagram for describing the dissolving states in a liquid.
[Fig. 4] Fig. 4 is a schematic configuration diagram of a T-UFB generating unit.
[Fig. 5] Fig. 5 is a diagrams for describing details of a heating element.
[Fig. 6] Fig. 6 is a diagrams for describing the states of film boiling on the heating element.
[Fig. 7] Fig. 7 is a diagrams illustrating the states of generation of UFBs caused by expansion of a film boiling bubble.
[Fig. 8] Fig. 8 is a diagrams illustrating the states of generation of UFBs caused by shrinkage of the film boiling bubble.
[Fig. 9] Fig. 9 is a diagrams illustrating the states of generation of UFBs caused by reheating of the liquid.
[Fig. 10] Fig. 10 is a diagrams illustrating the states of generation of UFBs caused by shock waves made by disappearance of the bubble generated by the film boiling.
[Fig. 11] Fig. 11 is a diagrams illustrating a configuration example of a post-processing unit.
[Fig. 12] Fig. 12 is a schematic view for describing states of flocculation and dispersion of T-UFBs.
[Fig. 13] Fig. 13 is a diagram illustrating a degerming effect in a UFB-containing liquid.
[Fig. 14] Fig. 14 is a schematic view illustrating a mechanism that Escherichia coli is killed by ozone UFBs.
[Fig. 15] Fig. 15 is a schematic view illustrating a mechanism that koji mold is killed by ozone UFBs.
[Fig. 16] Fig. 16 is a diagram illustrating a result of a purifying test.
[Fig. 17] Fig. 17 is a schematic view illustrating a mechanism that a solution containing heavy metal is purified.
[Fig. 18] Fig. 18 is a diagram illustrating a result of a detoxifying test.
[Fig. 19] Fig. 19 is a diagram illustrating a result of a cleaning test.
[Fig. 20] Fig. 20 is schematic views illustrating states of removing contamination by the UFB-containing liquid.
[Fig. 21] Fig. 21 is a schematic configuration view of a device for utilizing a UFB-containing liquid.

### Description of Embodiments

### [First Embodiment]

### «Configuration of UFB Generating Apparatus»

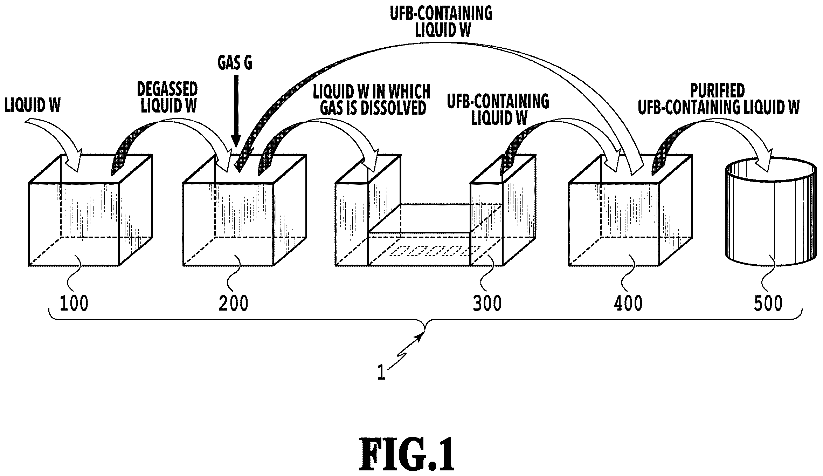

Fig. 1 is a diagram illustrating an example of a UFB generating apparatus applicable to the present embodiment. A UFB generating apparatus 1 of this embodiment includes a pre-processing unit 100, dissolving unit 200, a T-UFB generating unit 300, a post-processing unit 400, and a collecting unit 500. Each unit performs unique processing on a liquid W such as tap water supplied to the pre-processing unit 100 in the above order, and the thus-processed liquid W is collected as a T-UFB-containing liquid by the collecting unit 500. Functions and configurations of the units are described below.

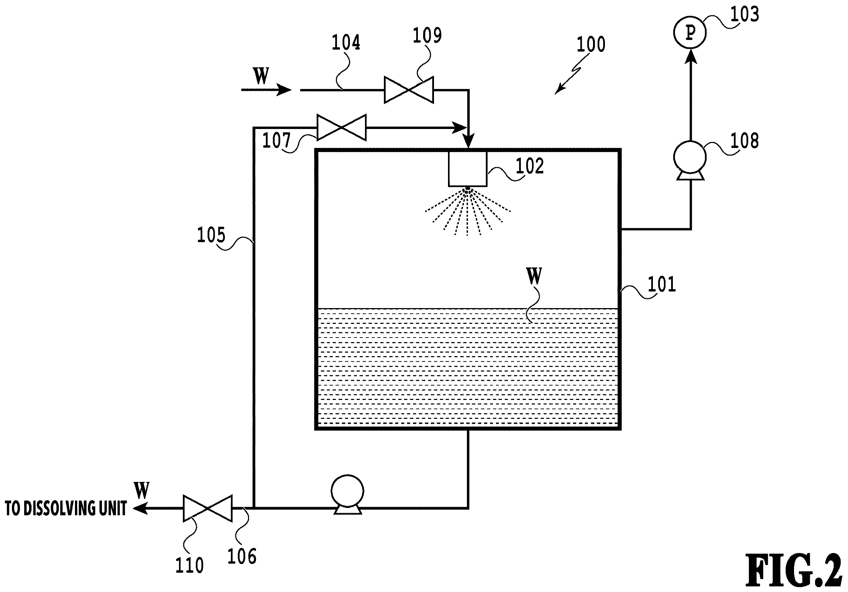

Fig. 2 is a schematic configuration diagram of the pre-processing unit 100. The pre-processing unit 100 of this embodiment performs a degassing treatment on the supplied liquid W. The pre-processing unit 100 mainly includes a degassing container 101, a shower head 102, a depressurizing pump 103, a liquid introduction passage 104, a liquid circulation passage 105, and a liquid discharge passage 106. For example, the liquid W such as tap water is supplied from the liquid introduction passage 104 to the degassing container 101 through a valve 109. In this process, the shower head 102 provided in the degassing container 101 sprays a mist of the liquid W in the degassing container 101. The shower head 102 is for prompting the gasification of the liquid W; however, a centrifugal and the like may be used instead as the mechanism for producing the gasification prompt effect.

When a certain amount of the liquid W is retained in the degassing container 101 and then the depressurizing pump 103 is activated with all the valves closed, alreadygasified gas components are discharged, and gasification and discharge of gas components dissolved in the liquid W are also prompted. In this process, the internal pressure of the degassing container 101 may be depressurized to around several hundreds to thousands of Pa (1.0 Torr to 10.0 Torr) while checking a manometer 108. The gases to be removed by the pre-processing unit 100 includes nitrogen, oxygen, argon, carbon dioxide, and so on, for example.

The above-described degassing processing can be repeatedly performed on the same liquid W by utilizing the liquid circulation passage 105. Specifically, the shower head 102 is operated with the valve 109 of the liquid introduction passage 104 and a valve 110 of the liquid discharge passage 106 closed and a valve 107 of the liquid circulation passage 105 opened. This allows the liquid W retained in the degassing container 101 and degassed once to be resprayed in the degassing container 101 from the shower head 102. In addition, with the depressurizing pump 103 operated, the gasification processing by the shower head 102 and the degassing processing by the depressurizing pump 103 are repeatedly performed on the same liquid W. Every time the above processing utilizing the liquid circulation passage 105 is performed repeatedly, it is possible to decrease the gas components contained in the liquid W in stages. Once the liquid W degassed to a desired purity is obtained, the liquid W is transferred to the dissolving unit 200 through the liquid discharge passage 106 with the valve 110 opened.

Fig. 2 illustrates the pre-processing unit 100 that depressurizes the gas part to gasify the solute; however, the method of degassing the solution is not limited thereto. For example, a heating and boiling method for boiling the liquid W to gasify the solute may be employed, or a film degassing method for increasing the interface between the liquid and the gas using hollow fibers. A SEPAREL series (produced by DIC corporation) is commercially supplied as the degassing module using the hollow fibers. The SEPAREL series uses poly(4-methylpentene-1) (PMP) for the raw material of the hollow fibers and is used for removing air bubbles from ink and the like mainly supplied for a piezo head. In addition, two or more of an evacuating method, the heating and boiling method, and the film degassing method may be used together.

With the above-described degassing processing performed as pre-processing, it is possible to increase the purity and the solubility of a desired gas with respect to the liquid W in the dissolving processing described later. Additionally, it is possible to increase the purity of desired UFBs contained in the liquid W in the T-UFB generating unit described later. That is, it is possible to efficiently generate a UFB-containing liquid (ultra-fine bubble-containing liquid) with high purity by providing the pre-processing unit 100 to precede the dissolving unit 200 and the T-UFB generating unit 300.

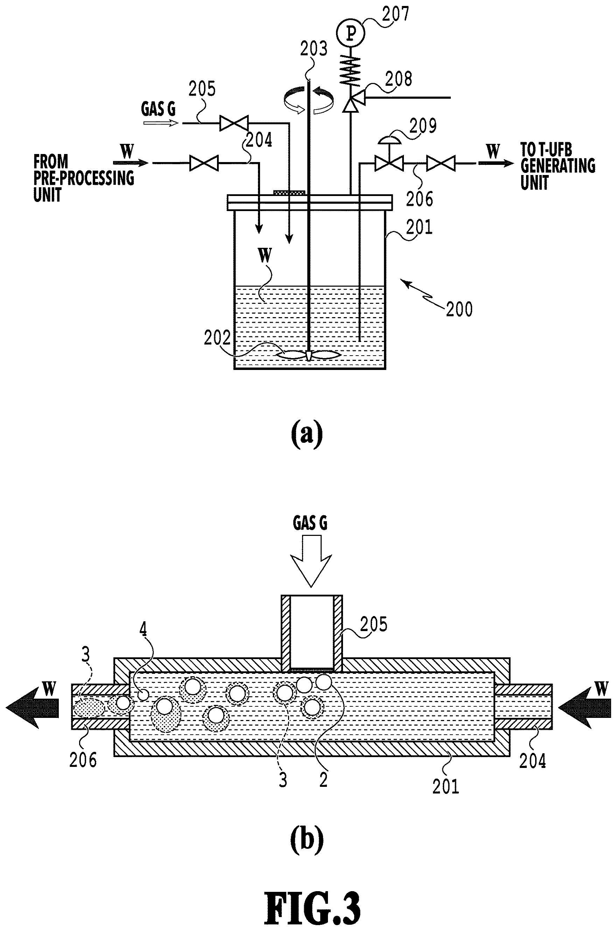

Figs. 3(a) and 3(b) are a schematic configuration diagram of the dissolving unit 200 and a diagram for describing the dissolving states in the liquid. The dissolving unit 200 is a unit for dissolving a desired gas into the liquid W supplied from the pre-processing unit 100. The dissolving unit 200 of this embodiment mainly includes a dissolving container 201, a rotation shaft 203 provided with a rotation plate 202, a liquid introduction passage 204, a gas introduction passage 205, a liquid discharge passage 206, and a pressurizing pump 207.

The liquid W supplied from the pre-processing unit 100 is supplied and retained into the dissolving container 201 through the liquid introduction passage 204. Meanwhile, a gas G is supplied to the dissolving container 201 through the gas introduction passage 205.

Once predetermined amounts of the liquid W and the gas G are retained in the dissolving container 201, the pressurizing pump 207 is activated to increase the internal pressure of the dissolving container 201 to about 0.5 MPa. A safety valve 208 is arranged between the pressurizing pump 207 and the dissolving container 201. With the rotation plate 202 in the liquid rotated via the rotation shaft 203, the gas G supplied to the dissolving container 201 is transformed into air bubbles, and the contact area between the gas G and the liquid W is increased to prompt the dissolution into the liquid W. This operation is continued until the solubility of the gas G reaches almost the maximum saturation solubility. In this case, a unit for decreasing the temperature of the liquid may be provided to dissolve the gas as much as possible. When the gas is with low solubility, it is also possible to increase the internal pressure of the dissolving container 201 to 0.5 MPa or higher. In this case, the material and the like of the container need to be the optimum for safety sake.

Once the liquid W in which the components of the gas G are dissolved at a desired concentration is obtained, the liquid W is discharged through the liquid discharge passage 206 and supplied to the T-UFB generating unit 300. In this process, a back-pressure valve 209 adjusts the flow pressure of the liquid W to prevent excessive increase of the pressure during the supplying.

Fig. 3(b) is a diagram schematically illustrating the dissolving states of the gas G put in the dissolving container 201. An air bubble 2 containing the components of the gas G put in the liquid W is dissolved from a portion in contact with the liquid W. The air bubble 2 thus shrinks gradually, and a gas-dissolved liquid 3 then appears around the air bubble 2. Since the air bubble 2 is affected by the buoyancy, the air bubble 2 may be moved to a position away from the center of the gas-dissolved liquid 3 or be separated out from the gas-dissolved liquid 3 to become a residual air bubble 4. Specifically, in the liquid W to be supplied to the T-UFB generating unit 300 through the liquid discharge passage 206, there is a mix of the air bubbles 2 surrounded by the gas-dissolved liquids 3 and the air bubbles 2 and the gas-dissolved liquids 3 separated from each other.

The gas-dissolved liquid 3 in Fig. 3(b) means "a region of the liquid W in which the dissolution concentration of the gas G mixed therein is relatively high." In the gas components actually dissolved in the liquid W in either case where the gas-dissolved liquid 3 is surrounding the air bubble 2 or separated from the air bubble 2, the concentration of the gas components in the center of the region is the highest, and the concentration is continuously decreased as away from the center. That is, although the region of the gas-dissolved liquid 3 is surrounded by a broken line in Fig. 3(b) for the sake of explanation, such a clear boundary does not actually exist. In addition, in the present embodiment, a gas that cannot be dissolved completely may be accepted to exist in the form of an air bubble in the liquid.

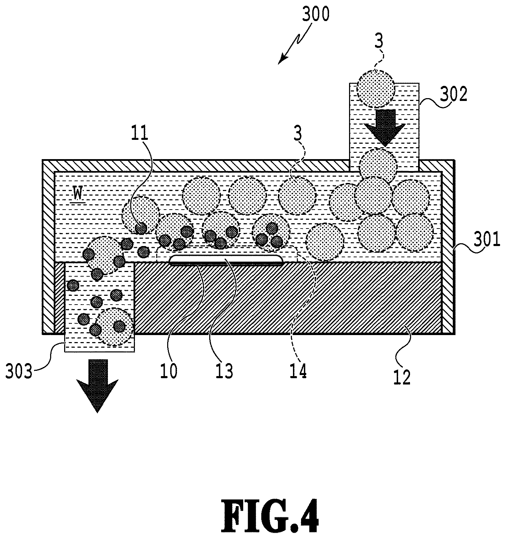

Fig. 4 is a schematic configuration diagram of the T-UFB generating unit 300. The T-UFB generating unit 300 mainly includes a chamber 301, a liquid introduction passage 302, and a liquid discharge passage 303. The flow from the liquid introduction passage 302 to the liquid discharge passage 303 through the chamber 301 is formed by a not-illustrated flow pump. Various pumps including a diaphragm pump, a gear pump, and a screw pump may be employed as the flow pump. The gas-dissolved liquid 3 of the gas G put by the dissolving unit 200 is mixed in the liquid W introduced from the liquid introduction passage 302.

An element substrate 12 provided with a heating element 10 is arranged on a bottom section of the chamber 301. With a predetermined voltage pulse applied to the heating element 10, a bubble 13 generated by the film boiling (hereinafter, also referred to as a film boiling bubble 13) is generated in a region in contact with the heating element 10. Then, an ultrafine bubble (UFB) 11 containing the gas G is generated caused by expansion and shrinkage of the film boiling bubble 13. As a result, a UFB-containing liquid W containing many UFBs 11 is discharged from the liquid discharge passage 303.

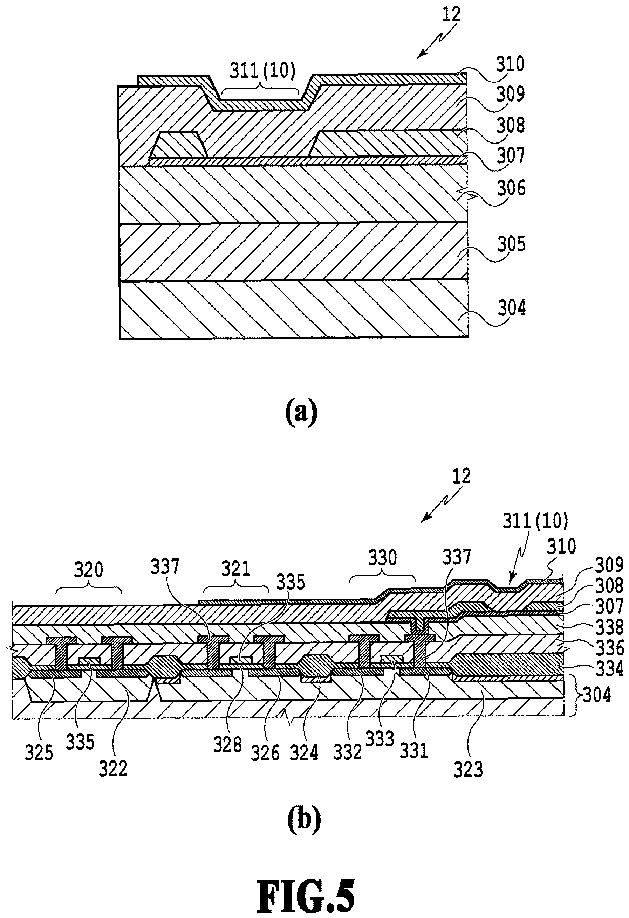

Figs. 5(a) and 5(b) are diagrams for illustrating a detailed configuration of the heating element 10. Fig. 5(a) illustrates a closeup view of the heating element 10, and Fig. 5(b) illustrates a cross-sectional view of a wider region of the element substrate 12 including the heating element 10.

As illustrated in Fig. 5(a), in the element substrate 12 of this embodiment, a thermal oxide film 305 as a heat-accumulating layer and an interlaminar film 306 also served as a heat-accumulating layer are laminated on a surface of a silicon substrate 304. An SiO₂ film or an SiN film may be used as the interlaminar film 306. A resistive layer 307 is formed on a surface of the interlaminar film 306, and a wiring 308 is partially formed on a surface of the resistive layer 307. An Al-alloy wiring of Al, Al-Si, Al-Cu, or the like may be used as the wiring 308. A protective layer 309 made of an SiO₂ film or an Si₃N₄ film is formed on surfaces of the wiring 308, the resistive layer 307, and the interlaminar film 306.

A cavitation-resistant film 310 for protecting the protective layer 309 from chemical and physical impacts due to the heat evolved by the resistive layer 307 is formed on a portion and around the portion on the surface of the protective layer 309, the portion corresponding to a heat-acting portion 311 that eventually becomes the heating element 10. A region on the surface of the resistive layer 307 in which the wiring 308 is not formed is the heat-acting portion 311 in which the resistive layer 307 evolves heat. The heating portion of the resistive layer 307 on which the wiring 308 is not formed functions as the heating element (heater) 10. As described above, the layers in the element substrate 12 are sequentially formed on the surface of the silicon substrate 304 by a semiconductor production technique, and the heat-acting portion 311 is thus provided on the silicon substrate 304.

The configuration illustrated in Fig. 5(a) is an example, and various other configurations are applicable. For example, a configuration in which the laminating order of the resistive layer 307 and the wiring 308 is opposite, and a configuration in which an electrode is connected to a lower surface of the resistive layer 307 (so-called a plug electrode configuration) are applicable. In other words, as described later, any configuration may be applied as long as the configuration allows the heat-acting portion 311 to heat the liquid for generating the film boiling in the liquid.

Fig. 5(b) is an example of a cross-sectional view of a region including a circuit connected to the wiring 308 in the element substrate 12. An N-type well region 322 and a P-type well region 323 are partially provided in a top layer of the silicon substrate 304, which is a P-type conductor. AP-MOS 320 is formed in the N-type well region 322 and an N-MOS 321 is formed in the P-type well region 323 by introduction and diffusion of impurities by the ion implantation and the like in the general MOS process.

The P-MOS 320 includes a source region 325 and a drain region 326 formed by partial introduction of N-type or P-type impurities in a top layer of the N-type well region 322, a gate wiring 335, and so on. The gate wiring 335 is deposited on a part of a top surface of the N-type well region 322 excluding the source region 325 and the drain region 326, with a gate insulation film 328 of several hundreds of Å in thickness interposed between the gate wiring 335 and the top surface of the N-type well region 322.

The N-MOS 321 includes the source region 325 and the drain region 326 formed by partial introduction of N-type or P-type impurities in a top layer of the P-type well region 323, the gate wiring 335, and so on. The gate wiring 335 is deposited on a part of a top surface of the P-type well region 323 excluding the source region 325 and the drain region 326, with the gate insulation film 328 of several hundreds of Å in thickness interposed between the gate wiring 335 and the top surface of the P-type well region 323. The gate wiring 335 is made of polysilicon of 3000 Å to 5000 Å in thickness deposited by the CVD method. A C-MOS logic is constructed with the P-MOS 320 and the N-MOS 321.

In the P-type well region 323, an N-MOS transistor 330 for driving an electrothermal conversion element (heating resistance element) is formed on a portion different from the portion including the N-MOS 321. The N-MOS transistor 330 includes a source region 332 and a drain region 331 partially provided in the top layer of the P-type well region 323 by the steps of introduction and diffusion of impurities, a gate wiring 333, and so on. The gate wiring 333 is deposited on a part of the top surface of the P-type well region 323 excluding the source region 332 and the drain region 331, with the gate insulation film 328 interposed between the gate wiring 333 and the top surface of the P-type well region 323.

In this example, the N-MOS transistor 330 is used as the transistor for driving the electrothermal conversion element. However, the transistor for driving is not limited to the N-MOS transistor 430, and any transistor may be used as long as the transistor has a capability of driving multiple electrothermal conversion elements individually and can implement the above-described fine configuration. Although the electrothermal conversion element and the transistor for driving the electrothermal conversion element are formed on the same substrate in this example, those may be formed on different substrates separately.

An oxide film separation region 324 is formed by field oxidation of 5000 Å to 10000 Å in thickness between the elements, such as between the P-MOS 320 and the N-MOS 321 and between the N-MOS 321 and the N-MOS transistor 330. The oxide film separation region 324 separates the elements. A portion of the oxide film separation region 324 corresponding to the heat-acting portion 311 functions as a heat-accumulating layer 334, which is the first layer on the silicon substrate 304.

An interlayer insulation film 336 including a PSG film, a BPSG film, or the like of about 7000 Å in thickness is formed by the CVD method on each surface of the elements such as the P-MOS 320, the N-MOS 321, and the N-MOS transistor 330. After the interlayer insulation film 336 is made flat by heat treatment, an Al electrode 337 as a first wiring layer is formed in a contact hole penetrating through the interlayer insulation film 336 and the gate insulation film 328. On surfaces of the interlayer insulation film 336 and the Al electrode 337, an interlayer insulation film 338 including an SiO₂ film of 10000 Å to 15000 Å in thickness is formed by a plasma CVD method. On the surface of the interlayer insulation film 338, a resistive layer 307 including a TaSiN film of about 500 Å in thickness is formed by a co-sputter method on portions corresponding to the heat-acting portion 311 and the N-MOS transistor 330. The resistive layer 307 is electrically connected with the Al electrode 337 near the drain region 331 via a throughhole formed in the interlayer insulation film 338. On the surface of the resistive layer 307, the wiring 308 of Al as a second wiring layer for a wiring to each electrothermal conversion element is formed. The protective layer 309 on the surfaces of the wiring 308, the resistive layer 307, and the interlayer insulation film 338 includes an SiN film of 3000 Å in thickness formed by the plasma CVD method. The cavitation-resistant film 310 deposited on the surface of the protective layer 309 includes a thin film of about 2000 Å in thickness, which is at least one metal selected from the group consisting of Ta, Fe, Ni, Cr, Ge, Ru, Zr, Ir, and the like. Various materials other than the above-described TaSiN such as TaN0.8, CrSiN, TaAl, WSiN, and the like can be applied as long as the material can generate the film boiling in the liquid.

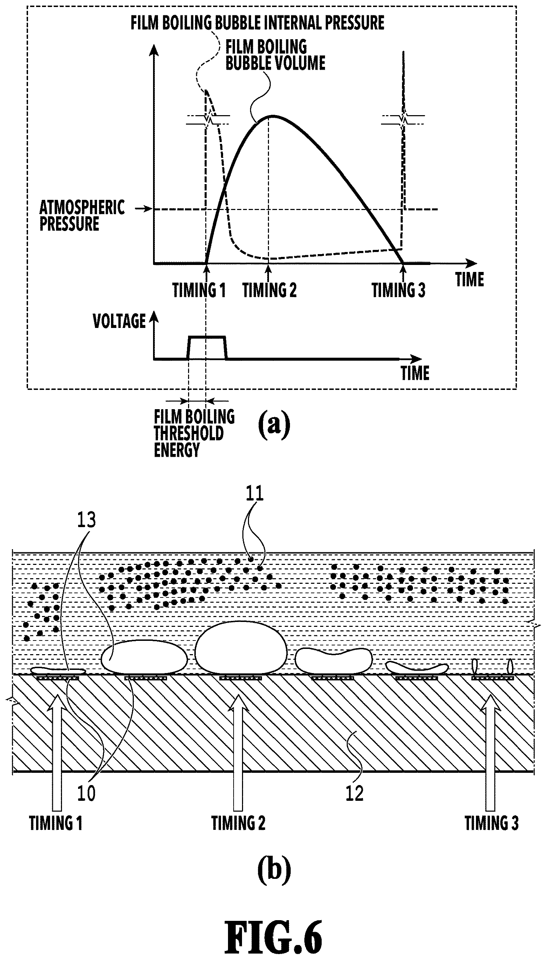

Figs. 6(a) and 6(b) are diagrams illustrating the states of the film boiling when a predetermined voltage pulse is applied to the heating element 10. In this case, the case of generating the film boiling under atmospheric pressure is described. In Fig. 6(a), the horizontal axis represents time. The vertical axis in the lower graph represents a voltage applied to the heating element 10, and the vertical axis in the upper graph represents the volume and the internal pressure of the film boiling bubble 13 generated by the film boiling. On the other hand, Fig. 6(b) illustrates the states of the film boiling bubble 13 in association with timings 1 to 3 shown in Fig. 6(a). Each of the states is described below in chronological order.

Before a voltage is applied to the heating element 10, the atmospheric pressure is substantially maintained in the chamber 301. Once a voltage is applied to the heating element 10, the film boiling is generated in the liquid in contact with the heating element 10, and a thus-generated air bubble (hereinafter, referred to as the film boiling bubble 13) is expanded by a high pressure acting from inside (timing 1). A bubbling pressure in this process is expected to be around 8 to 10 MPa, which is a value close to a saturation vapor pressure of water.

The time for applying a voltage (pulse width) is around 0.5 µsec to 10.0 µsec, and the film boiling bubble 13 is expanded by the inertia of the pressure obtained in timing 1 even after the voltage application. However, a negative pressure generated with the expansion is gradually increased inside the film boiling bubble 13, and the negative pressure acts in a direction to shrink the film boiling bubble 13. After a while, the volume of the film boiling bubble 13 becomes the maximum in timing 2 when the inertial force and the negative pressure are balanced, and thereafter the film boiling bubble 13 shrinks rapidly by the negative pressure.

In the disappearance of the film boiling bubble 13, the film boiling bubble 13 disappears not in the entire surface of the heating element 10 but in one or more extremely small regions. For this reason, on the heating element 10, further greater force than that in the bubbling in timing 1 is generated in the extremely small region in which the film boiling bubble 13 disappears (timing 3).

The generation, expansion, shrinkage, and disappearance of the film boiling bubble 13 as described above are repeated every time a voltage pulse is applied to the heating element 10, and new UFBs 11 are generated each time.

The states of generation of the UFBs 11 in each process of the generation, expansion, shrinkage, and disappearance of the film boiling bubble 13 are further described in detail.

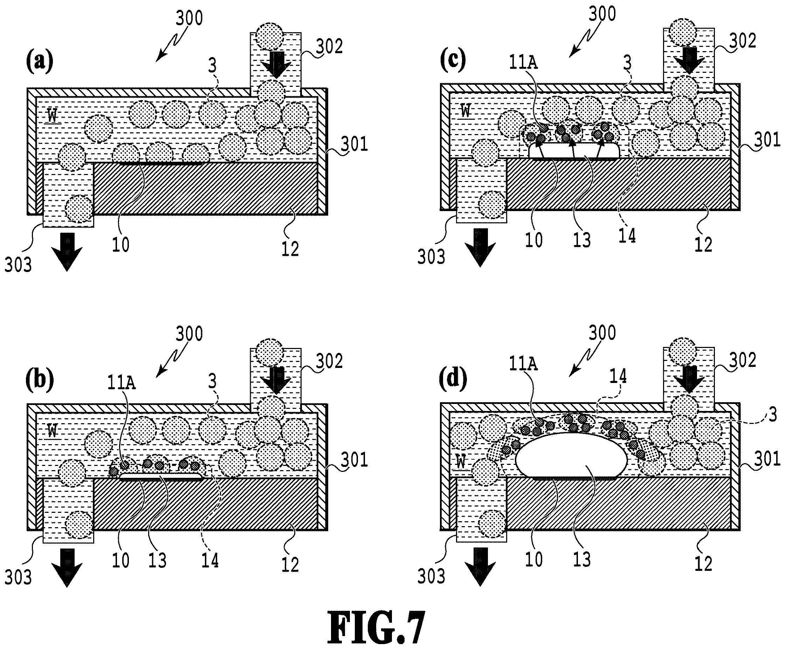

Figs. 7(a) to 7(d) are diagrams illustrating the states of generation of the UFBs 11 caused by the generation and the expansion of the film boiling bubble 13. Fig. 7(a) illustrates the state before the application of a voltage pulse to the heating element 10. The liquid W in which the gas-dissolved liquids 3 are mixed flows inside the chamber 301.

Fig. 7(b) illustrates the state where a voltage is applied to the heating element 10, and the film boiling bubble 13 is evenly generated in almost all over the region of the heating element 10 in contact with the liquid W. When a voltage is applied, the surface temperature of the heating element 10 rapidly increases at a speed of 10°C/µsec. The film boiling occurs at a time point when the temperature reaches almost 300°C, and the film boiling bubble 13 is thus generated.

Thereafter, the surface temperature of the heating element 10 keeps increasing to around 600 to 800°C during the pulse application, and the liquid around the film boiling bubble 13 is rapidly heated as well. In Fig. 7, a region of the liquid that is around the film boiling bubble 13 and to be rapidly heated is indicated as a not-yet-bubbling high temperature region 14. The gas-dissolved liquid 3 within the not-yet-bubbling high temperature region 14 exceeds the thermal dissolution limit and is precipitated to become the UFB. The thus-precipitated air bubbles have diameters of around 10 nm to 100 nm and large gas-liquid interface energy. Thus, the air bubbles float independently in the liquid W without disappearing in a short time. In this embodiment, the air bubbles generated by the thermal action during the expansion of the film boiling bubble 13 are called first UFBs 11A.

Fig. 7(c) illustrates the state where the film boiling bubble 13 is expanded. Even after the voltage pulse application to the heating element 10, the film boiling bubble 13 continues expansion by the inertia of the force obtained from the generation thereof, and the not-yet-bubbling high temperature region 14 is also moved and spread by the inertia. Specifically, in the process of the expansion of the film boiling bubble 13, the gas-dissolved liquid 3 within the not-yet-bubbling high temperature region 14 is precipitated as a new air bubble and becomes the first UFB 11A.

Fig. 7(d) illustrates the state where the film boiling bubble 13 has the maximum volume. As the film boiling bubble 13 is expanded by the inertia, the negative pressure inside the film boiling bubble 13 is gradually increased along with the expansion, and the negative pressure acts to shrink the film boiling bubble 13. At a time point when the negative pressure and the inertial force are balanced, the volume of the film boiling bubble 13 becomes the maximum, and then the shrinkage is started.

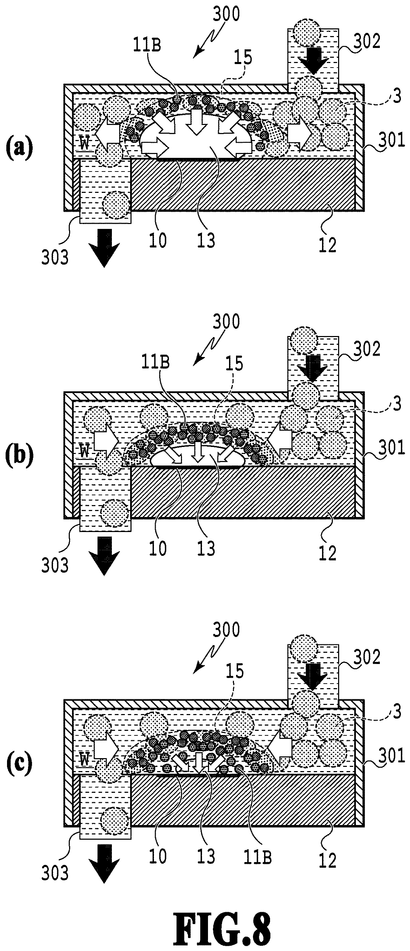

Figs. 8(a) to 8(c) are diagrams illustrating the states of generation of the UFBs 11 caused by the shrinkage of the film boiling bubble 13. Fig. 8(a) illustrates the state where the film boiling bubble 13 starts shrinking. Although the film boiling bubble 13 starts shrinking, the surrounding liquid W still has the inertial force in the expansion direction. Because of this, the inertial force acting in the direction of going away from the heating element 10 and the force going toward the heating element 10 caused by the shrinkage of the film boiling bubble 13 act in a surrounding region extremely close to the film boiling bubble 13, and the region is depressurized. The region is indicated in Fig. 8(a) as a not-yet-bubbling negative pressure region 15.

The gas-dissolved liquid 3 within the not-yet-bubbling negative pressure region 15 exceeds the pressure dissolution limit and is precipitated to become an air bubble. The thus-precipitated air bubbles have diameters of about 100 nm and thereafter float independently in the liquid W without disappearing in a short time. In this embodiment, the air bubbles precipitated by the pressure action during the shrinkage of the film boiling bubble 13 are called the second UFBs 11B.

Fig. 8(b) illustrates a process of the shrinkage of the film boiling bubble 13. The shrinking speed of the film boiling bubble 13 is accelerated by the negative pressure, and the not-yet-bubbling negative pressure region 15 is also moved along with the shrinkage of the film boiling bubble 13. Specifically, in the process of the shrinkage of the film boiling bubble 13, the gas-dissolved liquids 3 within a part over the not-yet-bubbling negative pressure region 15 are precipitated one after another and become the second UFBs 11B.

Fig. 8(c) illustrates the state immediately before the disappearance of the film boiling bubble 13. Although the moving speed of the surrounding liquid W is also increased by the accelerated shrinkage of the film boiling bubble 13, a pressure loss occurs due to a flow passage resistance in the chamber 301. As a result, the region occupied by the not-yet-bubbling negative pressure region 15 is further increased, and a number of the second UFBs 11B are generated.

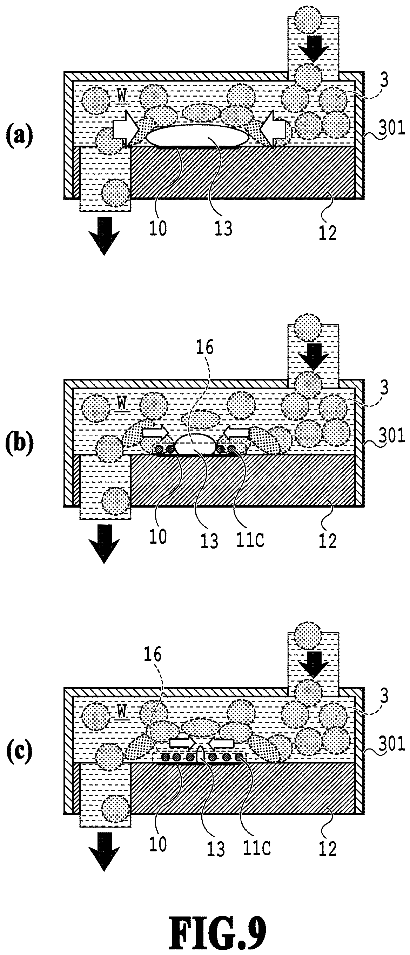

Figs. 9(a) to 9(c) are diagrams illustrating the states of generation of the UFBs by reheating of the liquid W during the shrinkage of the film boiling bubble 13. Fig. 9(a) illustrates the state where the surface of the heating element 10 is covered with the shrinking film boiling bubble 13.

Fig. 9(b) illustrates the state where the shrinkage of the film boiling bubble 13 has progressed, and a part of the surface of the heating element 10 comes in contact with the liquid W. In this state, there is heat left on the surface of the heating element 10, but the heat is not high enough to cause the film boiling even if the liquid W comes in contact with the surface. A region of the liquid to be heated by coming in contact with the surface of the heating element 10 is indicated in Fig. 9(b) as a not-yet-bubbling reheated region 16. Although the film boiling is not made, the gas-dissolved liquid 3 within the not-yet-bubbling reheated region 16 exceeds the thermal dissolution limit and is precipitated. In this embodiment, the air bubbles generated by the reheating of the liquid W during the shrinkage of the film boiling bubble 13 are called the third UFBs 11C.

Fig. 9(c) illustrates the state where the shrinkage of the film boiling bubble 13 has further progressed. The smaller the film boiling bubble 13, the greater the region of the heating element 10 in contact with the liquid W, and the third UFBs 11C are generated until the film boiling bubble 13 disappears.

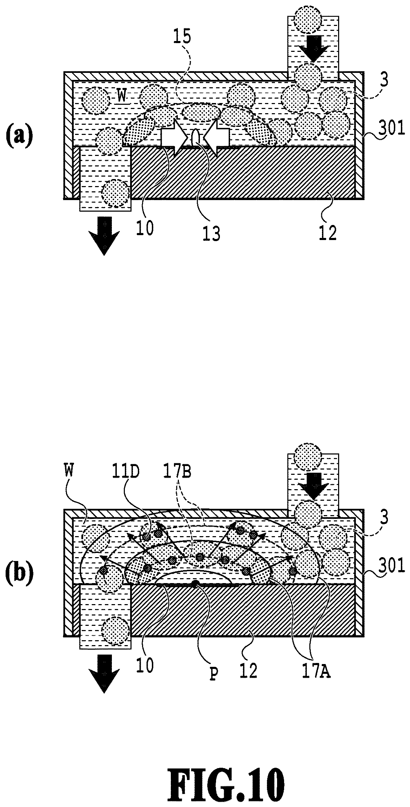

Figs. 10(a) and 10(b) are diagrams illustrating the states of generation of the UFBs caused by an impact from the disappearance of the film boiling bubble 13 generated by the film boiling (that is, a type of cavitation). Fig. 10(a) illustrates the state immediately before the disappearance of the film boiling bubble 13. In this state, the film boiling bubble 13 shrinks rapidly by the internal negative pressure, and the not-yet-bubbling negative pressure region 15 surrounds the film boiling bubble 13.

Fig. 10(b) illustrates the state immediately after the film boiling bubble 13 disappears at a point P. When the film boiling bubble 13 disappears, acoustic waves ripple concentrically from the point P as a starting point due to the impact of the disappearance. The acoustic wave is a collective term of an elastic wave that is propagated through anything regardless of gas, liquid, and solid. In this embodiment, coarse of the liquid W, which are a high pressure surface 17A and a low pressure surface 17B of the liquid W, are propagated alternately.

In this case, the gas-dissolved liquid 3 within the not-yet-bubbling negative pressure region 15 is resonated by the shock waves made by the disappearance of the film boiling bubble 13, and the gas-dissolved liquid 3 exceeds the pressure dissolution limit and the phase transition is made in timing when the low pressure surface 17B passes therethrough. Specifically, a number of air bubbles are precipitated in the not-yet-bubbling negative pressure region 15 simultaneously with the disappearance of the film boiling bubble 13. In this embodiment, the air bubbles generated by the shock waves made by the disappearance of the film boiling bubble 13 are called fourth UFBs 11D.

The fourth UFBs 11D generated by the shock waves made by the disappearance of the film boiling bubble 13 suddenly appear in an extremely short time (1 µS or less) in an extremely narrow thin film-shaped region. The diameter is sufficiently smaller than that of the first to third UFBs, and the gas-liquid interface energy is higher than that of the first to third UFBs. For this reason, it is considered that the fourth UFBs 11D have different characteristics from the first to third UFBs 11A to 11C and generate different effects.

Additionally, the fourth UFBs 11D are evenly generated in many parts of the region of the concentric sphere in which the shock waves are propagated, and the fourth UFBs 11D evenly exist in the chamber 301 from the generation thereof. Although many first to third UFBs already exist in the timing of the generation of the fourth UFBs 11D, the presence of the first to third UFBs does not affect the generation of the fourth UFBs 11D greatly. The first to third UFBs do not disappear due to the generation of the fourth UFBs 11D.

As described above, the UFBs 11 are generated in the multiple stages from the generation to the disappearance of the film boiling bubble 13 by the heat generation of the heating element 10. Although the above example illustrates the stages to the disappearance of the film boiling bubble 13, the way of generating the UFBs is not limited thereto. For example, with the generated film boiling bubble 13 communicating with the atmospheric air before the bubble disappearance, the UFBs can be generated also if the film boiling bubble 13 does not reach the exhaustion.

Next, remaining properties of the UFBs are described. The higher the temperature of the liquid, the lower the dissolution properties of the gas components, and the lower the temperature, the higher the dissolution properties of the gas components. In other words, the phase transition of the dissolved gas components is prompted and the generation of the UFBs becomes easier as the temperature of the liquid is higher. The temperature of the liquid and the solubility of the gas are in the inverse relationship, and the gas exceeding the saturation solubility is transformed into air bubbles and precipitated into the liquid as the liquid temperature increases.

Therefore, when the temperature of the liquid rapidly increases from normal temperature, the dissolution properties are decreased without stopping, and the generation of the UFBs starts. The thermal dissolution properties are decreased as the temperature increases, and a number of the UFBs are generated.

Conversely, when the temperature of the liquid decreases from normal temperature, the dissolution properties of the gas are increased, and the generated UFBs are more likely to be liquefied. However, the temperature is sufficiently lower than normal temperature. Additionally, since the once generated UFBs have a high internal pressure and large gas-liquid interface energy even when the temperature of the liquid decreases, it is highly unlikely that there is exerted a sufficiently high pressure to break such a gas-liquid interface. In other words, the once generated UFBs do not disappear easily as long as the liquid is stored at normal temperature and normal pressure.

In this embodiment, the first UFBs 11A described with Figs. 7(a) to 7(c) and the third UFBs 11C described with Figs. 9(a) to 9(c) can be described as UFBs that are generated by utilizing such thermal dissolution properties of gas.

On the other hand, in the relationship between the pressure and the dissolution properties of liquid, the higher the pressure of the liquid, the higher the dissolution properties of the gas, and the lower the pressure, the lower the dissolution properties. In other words, the phase transition to the gas of the gas-dissolved liquid dissolved in the liquid is prompted and the generation of the UFBs becomes easier as the pressure of the liquid is lower. Once the pressure of the liquid becomes lower than normal pressure, the dissolution properties are decreased without stopping, and the generation of the UFBs starts. The pressure dissolution properties are decreased as the pressure decreases, and a number of the UFBs are generated.

Conversely, when the pressure of the liquid increases to be higher than normal temperature, the dissolution properties of the gas are increased, and the generated UFBs are more likely to be liquefied. However, the pressure is sufficiently higher than the atmospheric pressure. Additionally, since the once generated UFBs have a high internal pressure and large gas-liquid interface energy even when the pressure of the liquid increases, it is highly unlikely that there is exerted a sufficiently high pressure to break such a gas-liquid interface. In other words, the once generated UFBs do not disappear easily as long as the liquid is stored at normal temperature and normal pressure.

In this embodiment, the second UFBs 11B described with Figs. 8(a) to 8(c) and the fourth UFBs 11D described with Figs. 10(a) to 10(c) can be described as UFBs that are generated by utilizing such pressure dissolution properties of gas.

Those first to fourth UFBs generated by different causes are described individually above; however, the above-described generation causes occur simultaneously with the event of the film boiling. Thus, at least two types of the first to the fourth UFBs may be generated at the same time, and these generation causes may cooperate to generate the UFBs. It should be noted that it is common for all the generation causes to be induced by the film boiling phenomenon. Hereinafter, in this specification, the method of generating the UFBs by utilizing the film boiling caused by the rapid heating as described above is referred to as a thermal-ultrafine bubble (T-UFB) generating method. Additionally, the UFBs generated by the T-UFB generating method are referred to as T-UFBs, and the liquid containing the T-UFBs generated by the T-UFB generating method is referred to as a T-UFB-containing liquid.

Almost all the air bubbles generated by the T-UFB generating method are 1.0 µm or less, and milli-bubbles and microbubbles are unlikely to be generated. That is, the T-UFB generating method allows efficient generation of the only UFBs. Additionally, the T-UFBs generated by the T-UFB generating method have larger gas-liquid interface energy than that of the UFBs generated by a conventional method, and the T-UFBs do not disappear easily as long as being stored at normal temperature and normal pressure. Moreover, even if new T-UFBs are generated by new film boiling, the already generated T-UFBs do not disappear due to the impact from the new generation. That is, it can be said that the number and the concentration of the T-UFBs contained in the T-UFB-containing liquid have the hysteresis properties depending on the number of times the film boiling is made in the T-UFB-containing liquid. In other words, it is possible to adjust the concentration of the T-UFBs contained in the T-UFB-containing liquid by controlling the number of the heating elements provided in the T-UFB generating unit 300 and the number of the voltage pulse application to the heating elements.

Reference to Fig. 1 is made again. Once the T-UFB-containing liquid W with a desired UFB concentration is generated in the T-UFB generating unit 300, the UFB-containing liquid W is supplied to the post-processing unit 400.

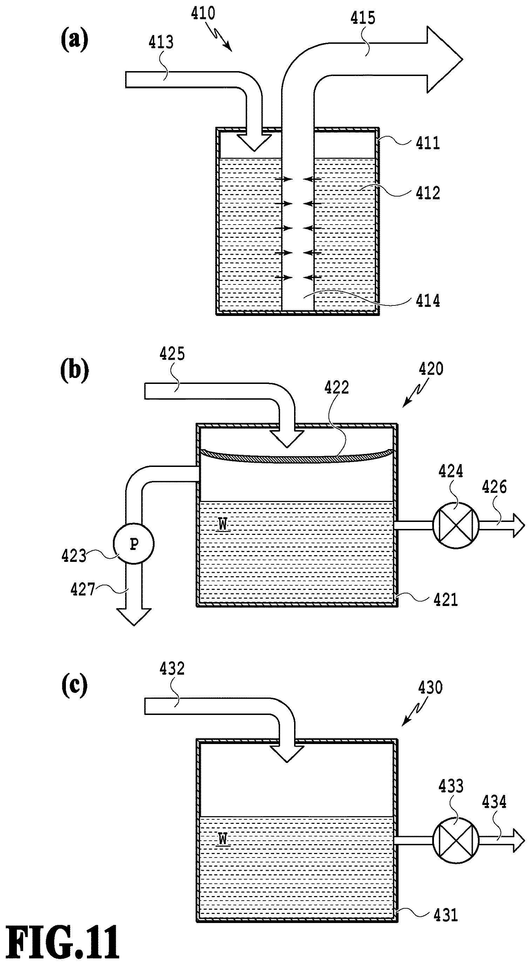

Figs. 11(a) to 11(c) are diagrams illustrating configuration examples of the post-processing unit 400 of this embodiment. The post-processing unit 400 of this embodiment removes impurities in the UFB-containing liquid W in stages in the order from inorganic ions, organic substances, and insoluble solid substances.

Fig. 11(a) illustrates a first post-processing mechanism 410 that removes the inorganic ions. The first post-processing mechanism 410 includes an exchange container 411, cation exchange resins 412, a liquid introduction passage 413, a collecting pipe 414, and a liquid discharge passage 415. The exchange container 411 stores the cation exchange resins 412. The UFB-containing liquid W generated by the T-UFB generating unit 300 is injected to the exchange container 411 through the liquid introduction passage 413 and absorbed into the cation exchange resins 412 such that the cations as the impurities are removed. Such impurities include metal materials peeled off from the element substrate 12 of the T-UFB generating unit 300, such as SiO₂, SiN, SiC, Ta, Al₂O₃, Ta₂O₅, and Ir.

The cation exchange resins 412 are synthetic resins in which a functional group (ion exchange group) is introduced in a high polymer matrix having a three-dimensional network, and the appearance of the synthetic resins are spherical particles of around 0.4 to 0.7 mm. A general high polymer matrix is the styrene-divinylbenzene copolymer, and the functional group may be that of methacrylic acid series and acrylic acid series, for example. However, the above material is an example. As long as the material can remove desired inorganic ions effectively, the above material can be changed to various materials. The UFB-containing liquid W absorbed in the cation exchange resins 412 to remove the inorganic ions is collected by the collecting pipe 414 and transferred to the next step through the liquid discharge passage 415.

Fig. 11(b) illustrates a second post-processing mechanism 420 that removes the organic substances. The second post-processing mechanism 420 includes a storage container 421, a filtration filter 422, a vacuum pump 423, a valve 424, a liquid introduction passage 425, a liquid discharge passage 426, and an air suction passage 427. Inside of the storage container 421 is divided into upper and lower two regions by the filtration filter 422. The liquid introduction passage 425 is connected to the upper region of the upper and lower two regions, and the air suction passage 427 and the liquid discharge passage 426 are connected to the lower region thereof. Once the vacuum pump 423 is driven with the valve 424 closed, the air in the storage container 421 is discharged through the air suction passage 427 to make the pressure inside the storage container 421 negative pressure, and the UFB-containing liquid W is thereafter introduced from the liquid introduction passage 425. Then, the UFB-containing liquid W from which the impurities are removed by the filtration filter 422 is retained into the storage container 421.

The impurities removed by the filtration filter 422 include organic materials that may be mixed at a tube or each unit, such as organic compounds including silicon, siloxane, and epoxy, for example. A filter film usable for the filtration filter 422 includes a filter of a sub-µm-mesh that can remove bacteria, and a filter of a nm-mesh that can remove virus.

After a certain amount of the UFB-containing liquid W is retained in the storage container 421, the vacuum pump 423 is stopped and the valve 424 is opened to transfer the T-UFB-containing liquid in the storage container 421 to the next step through the liquid discharge passage 426. Although the vacuum filtration method is employed as the method of removing the organic impurities herein, a gravity filtration method and a pressurized filtration can also be employed as the filtration method using a filter, for example.

Fig. 11(c) illustrates a third post-processing mechanism 430 that removes the insoluble solid substances. The third post-processing mechanism 430 includes a precipitation container 431, a liquid introduction passage 432, a valve 433, and a liquid discharge passage 434.

First, a predetermined amount of the UFB-containing liquid W is retained into the precipitation container 431 through the liquid introduction passage 432 with the valve 433 closed, and leaving it for a while. Meanwhile, the solid substances in the UFB-containing liquid W are precipitated onto the bottom of the precipitation container 431 by gravity. Among the bubbles in the UFB-containing liquid, relatively large bubbles such as microbubbles are raised to the liquid surface by the buoyancy and also removed from the UFB-containing liquid. After a lapse of sufficient time, the valve 433 is opened, and the UFB-containing liquid W from which the solid substances and large bubbles are removed is transferred to the collecting unit 500 through the liquid discharge passage 434.

Reference to Fig. 1 is made again. The T-UFB-containing liquid W from which the impurities are removed by the post-processing unit 400 may be directly transferred to the collecting unit 500 or may be put back to the dissolving unit 200 again. In the latter case, the gas dissolution concentration of the T-UFB-containing liquid W that is decreased due to the generation of the T-UFBs can be compensated to the saturated state again by the dissolving unit 200. If new T-UFBs are generated by the T-UFB generating unit 300 after the compensation, it is possible to further increase the concentration of the UFBs contained in the T-UFB-containing liquid with the above-described properties. That is, it is possible to increase the concentration of the contained UFBs by the number of circulations through the dissolving unit 200, the T-UFB generating unit 300, and the post-processing unit 400, and it is possible to transfer the UFB-containing liquid W to the collecting unit 500 after a predetermined concentration of the contained UFBs is obtained.

Here, an effect of putting back the generated T-UFB-containing liquid W to the dissolving unit 200 again is simply described in accordance with details of specific testing performed by the present inventors. First, in the T-UFB generating unit 300, 10000 pieces of the heating elements 10 were arranged on the element substrate 12. Industrial pure water was used as the liquid W and was flowed in the chamber 301 of the T-UFB generating unit 300 at a flow rate of 1.0 liter/hour. I this state, a voltage pulse with a voltage of 24 V and a pulse width of 1.0 µs was applied at a driving frequency of 10 KHz to the individual heating elements.

In a case where the generated T-UFB-containing liquid W was collected by the collecting unit 500 without putting back to the dissolving unit 200, that is, in a case where the number of circulation was one time, 3.6 billion pieces per mL of the UFBs were confirmed in the T-UFB-containing liquid W collected by the collecting unit 500. On the other hand, in a case where the operation of putting back the T-UFB-containing liquid W to the dissolving unit 200 was performed ten times, that is, in a case where the number of circulation was ten times, 36 billion pieces per mL of the UFBs were confirmed in the T-UFB-containing liquid W collected by the collecting unit 500. That is, it was confirmed that the UFB-containing concentration is increased in the proportion of the number of circulation. The number density of the UFBs as described above was obtained by counting the UFBs smaller than 1.0 µm in diameter contained in the UFB-containing liquid W of a predetermined volume by using a measuring instrument (model number SALD-7500) manufactured by SHIMADZU CORPORATION.

The collecting unit 500 collects and preserves the UFB-containing liquid W transferred from the post-processing unit 400. The T-UFB-containing liquid collected by the collecting unit 500 is a UFB-containing liquid with high purity from which various impurities are removed.

In the collecting unit 500, the UFB-containing liquid W may be classified by the size of the T-UFBs by performing some stages of filtration processing. Since it is expected that the temperature of the T-UFB-containing liquid W obtained by the T-UFB method is higher than normal temperature, the collecting unit 500 may be provided with a cooling unit. The cooling unit may be provided to a part of the post-processing unit 400.

The schematic description of the UFB generating apparatus 1 is given above; however, it is needless to say that the illustrated multiple units can be changed, and not all of them need to be prepared. Depending on the type of the liquid W and the gas G to be used and the intended use of the T-UFB-containing liquid to be generated, a part of the above-described units may be omitted, or another unit other than the above-described units may be added.

For example, when the gas to be contained by the UFBs is the atmospheric air, the pre-processing unit 100 and the dissolving unit 200 can be omitted. On the other hand, when multiple kinds of gases are desired to be contained by the UFBs, another dissolving unit 200 may be added.

The functions of some units illustrated in Fig. 1 can be integrated into a single unit. For example, the dissolving unit 200 and the T-UFB generating unit 300 can be integrated by arranging the heating element 10 in the dissolving container 201 illustrated in Figs. 3(a) and 3(b). Specifically, an electrode type T-UFB module is disposed in a gas dissolving container (high-pressure chamber), and multiple heaters arranged in the module are driven to make film boiling. Such a configuration allows a single unit to generate T-UFBs containing a gas while dissolving the gas therein. In this case, with the T-UFB module arranged on a base of the gas dissolving container, a Marangoni flow occurs due to the heat generated by the heaters, and the liquid in the container can be agitated to some extent without providing a circulating and agitating unit.

The removing units for removing the impurities as described in Figs. 11(a) to 11(c) may be provided, a part of a pre-processing unit, upstream of the T-UFB generating unit 300 or may be provided both upstream and downstream thereof. When the liquid to be supplied to the UFB generating apparatus is tap water, rain water, contaminated water, or the like, there may be included organic and inorganic impurities in the liquid. If such a liquid W including the impurities is supplied to the T-UFB generating unit 300, there is a risk of deteriorating the heating element 10 and inducing the salting-out phenomenon. With the mechanisms as illustrated in Figs. 11(a) to 11(c) provided upstream of the T-UFB generating unit 300, it is possible to remove the above-described impurities previously and to more efficiently generate a UFB-containing liquid with higher purity.

Particularly, in a case where an impurity removing unit using an ion-exchange resin illustrated in Fig. 11A is provided in the pre-processing unit, arrangement of an anion-exchange resin contributes to efficient generation of T-UFB water. This is because it has been confirmed that the ultra-fine bubbles generated by the T-UFB generating unit 300 have a negative charge. Accordingly, T-UFB water with high purity can be generated by removing the impurities having the same negative charges in the pre-processing unit. As the anion-exchange resin used herein, both the strongly basic anion-exchange resin having quaternary ammonium group and weakly basic anion-exchange resin having primary to tertiary amine group are appropriate. Which of these is appropriate depends on the type of the liquid to be used. Usually, in a case of using tap water, pure water, or the like as the liquid, the function of removing the impurities can be fulfilled sufficiently by using only the latter weakly basic anion-exchange resin.

### «Liquid and Gas Usable for T-UFB-Containing Liquid»

Here is described the liquid W that is usable for generating a T-UFB-containing liquid. The liquid W usable in the present embodiment includes, for example, pure water, ion-exchange water, distilled water, physiologically active water, magnetic active water, lotion, tap water, sea water, river water, clean and sewage water, lake water, underground water, rain water, and so on. Additionally, a mixed liquid containing the above liquid and the like is also usable. Moreover, a mixed solvent containing water and a water-soluble organic solvent can also be used. The water-soluble organic solvent to be used by being mixed with water is not particularly limited; however, the followings can be a specific example thereof. An alkyl alcohol group of the carbon number of 1 to 4 including methyl alcohol, ethyl alcohol, n-propyl alcohol, isopropyl alcohol, n-butyl alcohol, sec-butyl alcohol, tert-butyl alcohol, and so on. An amide group including N-methyl-2-pyrrolidone, 2-pyrrolidone, 1,3-dimethyl-2-imidazolidinone, N,N-dimethylformamide, N,N-dimethylacetamide, and so on. A keton or ketoalcohol group including acetone, diacetone alcohol, and so on. A cyclic ether group including tetrahydrofuran, dioxane, and so on. A glycol group including ethylene glycol, 1,2-propylene glycol, 1,3-propylene glycol, 1,2-butanediol, 1,3-butanediol, 1,4-butanediol, 1,5-pentanediol, 1,2-hexanediol, 1,6-hexanediol, 3-methyl-1,5-pentanediol, diethylene glycol, triethylene glycol, thiodiglycol, and so on. A group of lower alkyl ether of polyhydric alcohol including ethylene glycol monomethyl ether, ethylene glycol monoethyl ether, ethylene glycol monobutyl ether, diethylene glycol monomethyl ether, diethylene glycol monoethyl ether, diethylene glycol monobutyl ether, triethylene glycol monomethyl ether, triethylene glycol monoethyl ether, triethylene glycol monobutyl ether, and so on. A polyalkylene glycol group including polyethylene glycol, polypropylene glycol, and so on. A triol group including glycerin, 1,2,6-hexanetriol, trimethylolpropane, and so on. These water-soluble organic solvents may be used individually, or two or more of them may be used together.

A gas component that can be introduced in the dissolving unit 200 includes, for example, hydrogen, helium, oxygen, nitrogen, methane, fluorine, neon, carbon dioxide, ozone, argon, chlorine, ethane, propane, air, and so on. Additionally, the gas component may be a mixed gas containing some of the above. Moreover, it is unnecessary for the dissolving unit 200 to dissolve a substance in a gas state, and the dissolving unit 200 may fuse a liquid or a solid containing desired components into the liquid W. The dissolution in this case may be spontaneous dissolution, dissolution caused by pressure application, or dissolution with hydration, ionization, and chemical reaction caused by electrolytic dissociation.

### <<Specific Example of Case of Using Ozone Gas>>

As a specific example, here is described a case of using an ozone gas as a gas component. First, a generating method for an ozone gas includes an electric discharge method, an electrolytic method, and an ultraviolet lamp method. The above methods are described below in sequence.

### (1) Electric Discharge Method

The electric discharge method includes a silent electric discharge method and a surface electric discharge method. In the silent electric discharge method, an alternating-current high voltage is applied while an oxygen-containing gas is flowed between a pair of electrodes arranged in the form of parallel flat plates or coaxial cylinders. With this, discharge occurs in the oxygen-containing gas, and an ozone gas is generated. One of or both the surfaces of the pair of electrodes need to be covered with a dielectric such as glass. The discharge occurs in a gas (air or oxygen) in association with alternate variation, positively and negatively, of charges on the surface of the dielectric.

On the other hand, in the surface electric discharge method, a surface of a flat surface-shaped electrode is covered with a dielectric such as ceramics, and a linear electrode is arranged on the surface of the dielectric. Then, an alternating-current high voltage is applied between the flat plate-shaped electrode and the linear electrode. With this, discharge occurs on the surface of the dielectric, and ozone gas is generated.

### (2) Electrolytic Method

A pair of electrodes with an electrolyte membrane arranged therebetween are arranged in water, and a direct-current voltage is applied between the two electrodes. With this, electrolysis of the water occurs, and an ozone gas is generated with oxygen on the oxygen generation side. An ozone generator being practically used includes porous titanium having a platinum catalyst layer on a cathode, porous titanium having a lead dioxide catalyst layer on an anode, one using a perfluorosulfonic acid cation-exchange membrane as an electrolyte membrane, and the like. According to the present apparatus, highly concentrated ozone of 20% by weight or greater can be generated.

### (3) Ultraviolet Lamp Method

An ozone gas is generated by exposing ultraviolet to the air and the like by using a similar principle as that of how the ozone layer of Earth is created. Usually, a mercury lamp is used as an ultraviolet lamp.

Note that, in a case of using an ozone gas as the gas component, an ozone gas generating unit employing the methods (1) to (3) described above may be additionally added to the UFB generating apparatus 1 in Fig. 1.

Next, a dissolving method for the generated ozone gas is described. A method appropriate for dissolving an ozone gas into the liquid W includes an "air bubble dissolution method", a "membrane contactor dissolution method", and a "filled-layer dissolution method" in addition to the pressurized dissolution method illustrated in Figs. 3(a) and 3(b). The above three methods are compared with each other and described below in sequence.

### (i) Air Bubble Dissolution Method

This is a method of mixing an ozone gas into the liquid W as bubbles and flowing the ozone gas with the liquid W to dissolve. For example, there are a bubbling method in which an ozone gas is blown from a lower portion of a container retaining the liquid W, an ejector method in which a narrow portion is provided in a part of a pipe through which the liquid W flows and an ozone gas is blown into the narrow portion, a method of agitating the liquid W and an ozone gas by a pump, and the like. The air bubble dissolution method is a relatively compact dissolution method and is used in a water treatment plant and the like.

### (ii) Membrane Contactor Dissolution Method

This is a method of absorbing and dissolving an ozone gas into the liquid W by flowing the liquid W through a porous Teflon (registered trademark) membrane while the ozone gas is flowed through the outside.

### (iii) Filled-Layer Dissolution Method

This is a method of dissolving an ozone gas into the liquid W in a filled-layer by making counterflow of the ozone gas and the liquid by flowing the liquid W from the top of the filled-layer while flowing the ozone gas from the bottom.

Note that, in a case of employing the methods (i) to (iii) described above, the dissolving unit 200 of the UFB generating apparatus 1 may be changed from the one with the configuration illustrated in Figs. 3(a) and 3(b) to the one with the configuration employing any one of the methods (i) to (iii).

Particularly, in terms of the severe toxicity, purchase and usage of an ozone gas with high purity with a gas cylinder are limited unless a special environment is prepared. For this reason, it is difficult to generate ozone microbubbles and ozone ultra-fine bubbles by conventional generating methods for microbubbles or ultra-fine bubbles by gas introduction (for example, a Venturi method, a swirl flow method, a pressurized dissolution method, and so on).

On the other hand, as a method of generating ozone dissolving water, a method of generating ozone from oxygen supplied by the above-described electric discharge method, electrolytic method, or ultraviolet lamp method and dissolving into the water concurrently is useful from the points of the safety and the handleability.

However, in cases of a cavitation method and the like, although it is possible to generate ozone ultra-fine bubbles by using the ozone dissolving water, there are still problems such as an increase in size of the apparatus and the difficulty in increasing the concentration of the ozone ultra-fine bubbles.

In contrast, the T-UFB generating method can be said to be better than the other generating methods in that the apparatus can be relatively small in size, and highly concentrated ozone ultra-fine bubbles can be generated from the ozone dissolving water.

### <<Effects of T-UFB Generating Method>>

Next, the characteristics and the effects of the above-described T-UFB generating method are described by comparing with a conventional UFB generating method. For example, in a conventional air bubble generating apparatus as represented by the Venturi method, a mechanical depressurizing structure such as a depressurizing nozzle is provided in a part of a flow passage. A liquid is flowed at a predetermined pressure to pass through the depressurizing structure, and thus air bubbles of various sizes are generated in a downstream region of the depressurizing structure.

In this case, among the generated air bubbles, since the bubbles of relatively large size such as milli-bubbles and microbubbles are affected by the buoyancy, such bubbles rise to the liquid surface and disappear in time. Additionally, even the UFBs that are not affected by the buoyancy disappear with the milli-bubbles and microbubbles since the gas-liquid interface energy of the UFBs is not very large. Moreover, even if the above-described depressurizing structures are arranged in series, and the same liquid is flowed through the depressurizing structures repeatedly, it is impossible to store for a long period of time the UFBs of the number corresponding to the number of repetitions. In other words, it has been difficult for the UFB-containing liquid generated by the conventional UFB generating method to maintain the concentration of the contained UFBs at a predetermined value for a long period of time.

In contrast, in the T-UFB generating method of the present embodiment using the film boiling, a rapid temperature change from normal temperature to about 300°C and a rapid pressure change from normal pressure to around a several megapascal occur locally in a part extremely close to the heating element. The heating element is a rectangular shape having one side of around several tens to hundreds of µm. It is around 1/10 to 1/1000 of the size of a conventional UFB generator. In addition, with the gas-dissolved liquid within an extremely thin film region of the film boiling bubble surface exceeding the thermal dissolution limit or the pressure dissolution limit instantaneously (in an extremely short time equal to or under microseconds), the phase transition occurs and the gas-dissolved liquid is precipitated as the UFBs. In this case, the bubbles of relatively large size such as milli-bubbles and microbubbles are hardly generated, and the liquid contains the UFBs of about 100 nm in diameter with extremely high purity. Moreover, since the T-UFBs generated in this way have sufficiently large gas-liquid interface energy, the T-UFBs are not broken easily under the normal environment and can be stored for a long period of time.

Particularly, in the present embodiment using the film boiling phenomenon that can form a gas interface locally in the liquid, an interface can be formed in a part of the liquid without affecting the entire liquid region, and a region accordingly affected by heat and pressure can be within an extremely local range. As a result, it is possible to stably generate desired UFBs. Additionally, with further more conditions for generating the UFBs applied to the generated liquid by circulating the liquid, it is possible to additionally generate new UFBs with small effects on the already-made UFBs. As a result, it is possible to produce a UFB liquid of a desired size and concentration relatively easily.

Furthermore, since the T-UFB generating method has the above-described hysteresis properties, the contained concentration can be increased to a desired concentration while keeping the high purity. In other words, according to the T-UFB generating method, it is possible to efficiently generate a long-time storable UFB-containing liquid with high purity and high concentration.

### <<Behavior of T-UFBs>>

According to the studies by the present inventors, it has been confirmed that the T-UFBs generated by the T-UFB generating method form a floc after being generated. Additionally, it has been also confirmed that such a floc is able to be dispersed by vibration applied.

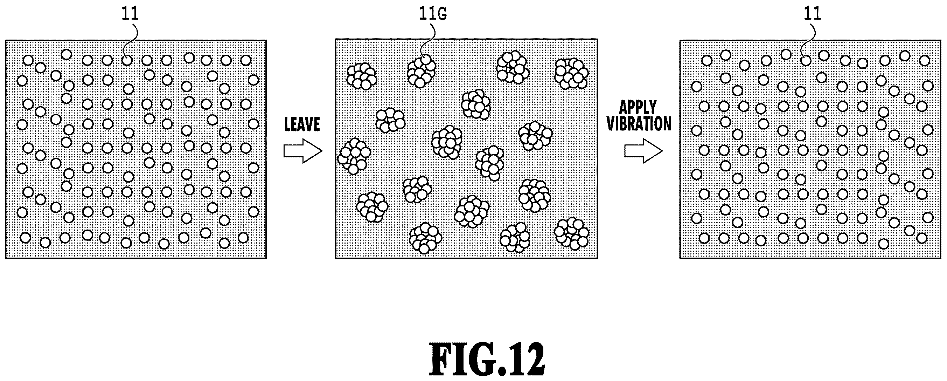

Fig. 12 is a schematic view for describing states of flocculation and dispersion of T-UFBs. The individual UFBs 11 generated by the T-UFB method have a zeta potential of ±0 to -30 mV and are fine bubbles of 100 nm or less in diameter that tend to be flocculated with the other. Accordingly, in the UFB-containing liquid generated by the T-UFB generating method, the formation of a floc 11G is started at a relatively early stage after the UFB generation.

As the number of the UFBs 11 contained in the floc 11G is greater, the negative charge that the floc 11G has is greater. Additionally, once the diameter of the floc 11G becomes around 100 to 200 nm, the zeta potential that the floc has becomes around -60 to -100 mV, and the flocs 11G repel each other due to electrostatic force. That is, in the T-UFB-containing liquid in which the UFBs are generated by the T-UFB generating method and after being left for a while, multiple flocs 11G of a size around 100 to 200 nm are stable in a state of dispersing and floating in the liquid with Brownian motion. Unlike microbubbles, the floc 11G does not rise in the liquid and separate into a gas at a gas-liquid interface and does not make cavitation destruction, and has similar characteristics as the UFBs 11 that float individually.

However, a state in which the UFBs 11 float by the unit of the floc 11G is a state in which the number of the UFBs is smaller than the number of the UFBs 11 in actuality, and an effect of the existence of many UFBs with high dispersibility is reduced. Under such circumstance, according to the studies by the present inventors, it has been confirmed that the floc 11G in a stable state can be dispersed into the individual UFBs 11 by applying predetermined vibration to the T-UFB-containing liquid. That is, it is expected that various effects that the UFB-containing liquid has are further improved by applying vibration to the T-UFB-containing liquid before using in any application.

Note that, the vibration number of the above-described vibration is able to be changed depending on a state as a matter of course. According to the studies by the present inventors, it has been confirmed that the floc 11G can be dispersed into the individual UFBs 11 with ultrasonic waves included in a KHz band to an MKHz band. Additionally, in order to suppress disappearance of the UFBs due to vibration while applying vibration to the liquid in which a flocculated matter containing the UFBs floats, it is preferable to adjust the frequency, output, and radiation time of the ultrasonic waves. Specifically, in the dispersing step, it is preferable to apply vibration to the liquid by irradiating the liquid with ultrasonic waves of the frequency of 1.0 MHz or less and the output of 50 W or less for 1 to 15 minutes.

### <<Specific Utility of T-UFB-Containing Liquid>>

Next, a utilizing example of the above-described T-UFB-containing liquid is specifically described using some examples.

### (Example 1)

In the present example, here is described an example of using the T-UFB-containing liquid generated by the T-UFB generating method as a liquid that degerms or kills bacteria and molds (hereinafter, referred to as a degerming liquid).

In the present example, something in which an ozone gas is dissolved by the dissolving unit 200, T-UFBs are generated by the T-UFB generating unit 300, and additionally ultrasonic waves are applied as post-processing in the UFB generating apparatus 1 described in Fig. 1 is used as a degerming liquid. Specifically, first, an ozone gas is dissolved by the dissolving unit 200, and a liquid in which the ozone concentration is 1300 ppm and the oxygen concentration is 9.9 ppm is generated. Next, T-UFBs are generated by the T-UFB generating unit 300, and a UFB concentration of 4.4 billion pieces/mL is obtained. Additionally, an ultrasonic horn is inserted into the obtained liquid, and ultrasonic waves are oscillated for 30 seconds at 300 kHz and 500 W. The liquid obtained by the above steps is the degerming liquid of the present example.

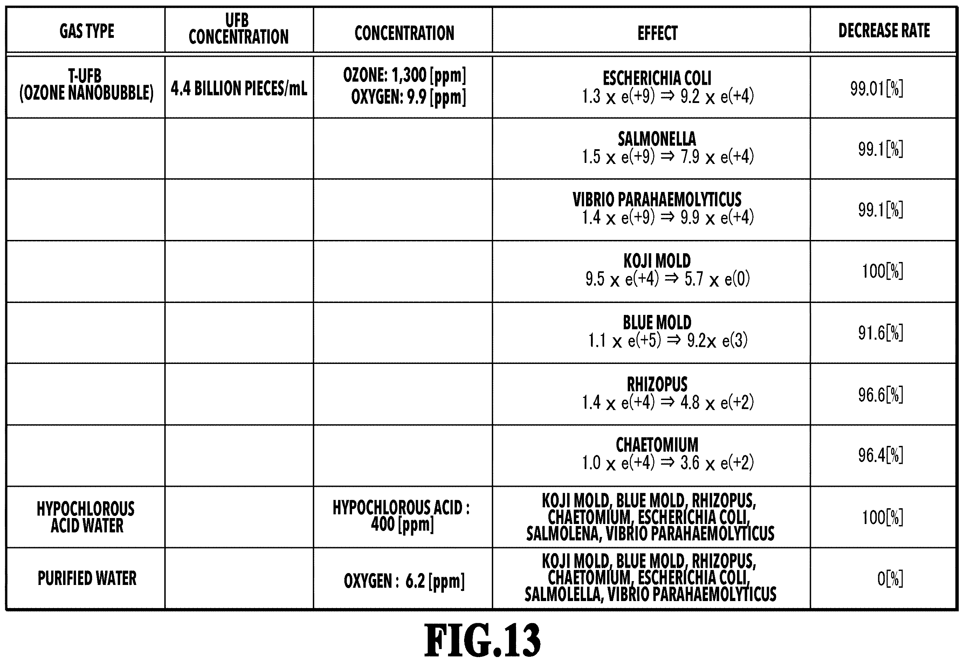

Fig. 13 is a diagram illustrating a degerming effect in the degerming liquid of the present example. As a sample of an evaluation target, bacteria and a mold were prepared. As a sample of bacteria, Escherichia coli, salmonella, and Vibrio parahaemolyticus that are "Gram-negative bacilli" were used. As a sample of a mold, koji mold, blue mold, rhizopus, and Chaetomium were used. In Fig. 13, a case of using hypochlorous acid water of 400 ppm and a case of using purified water as the degerming liquid are also indicated as comparative examples.

Here is simply described classification of bacteria. Usually, bacteria are roughly classified into two based on a structure of an outer wall of a cell. One is "Gram-positive bacteria" having a relatively thick and hard outer wall, and the other is "Gram-negative bacteria" having a relatively thin and fragile outer membrane. On the other hand, conventionally, bacteria are also classified based on the apparent shape such as "bacilli" in a long shape and "cocci" in a circular shape. Accordingly, bacteria can be classified into "Gram-positive bacilli", "Gram-positive cocci", "Gram-negative bacilli", "Gram-negative cocci", and the like based on the combinations of the above. Escherichia coli, salmonella, and Vibrio parahaemolyticus used as a sample of the present example are included in "Gram-negative bacilli" and are representative bacteria of Gram-negative bacilli. On the other hand, bacillus subtilis natto is known as a representative of "Gram-positive bacilli".

On the other hand, a "mold" is a common name of fungi over multiple classifying genres and is a term indicating a figure of some of fungi, or a common name of a settlement (colony) of tiny organisms observed with the naked eye that is seen as a state like a figure of some of fungi. Accordingly, there exists a "mold" with various lifestyles. The blue mold, koji mold, Chaetomium, and rhizopus used as a sample in the present example are molds like, as one might say, weeds that appear quickly in an artificial environment.

A testing method for confirming an effect of the degerming liquid of the present example is described below. First, a fungus body suspension of each bacterium or mold was put in contact with each degerming liquid for 72 hours. Then, a diluted solution diluted 10e(+6)-fold from the liquid after being in contact was created, and 100 µL out of the diluted solution was applied to a plate. Additionally, the plate subjected to the application was cultured, and the number of fungi was measured. With the above test, results illustrated in Fig. 13 were obtained. As can be seen in Fig. 13, in a case of using the degerming liquid of the present example, the degerming effect of 98% or more in all the bacteria samples and 90% or more in all the mold samples could be confirmed.

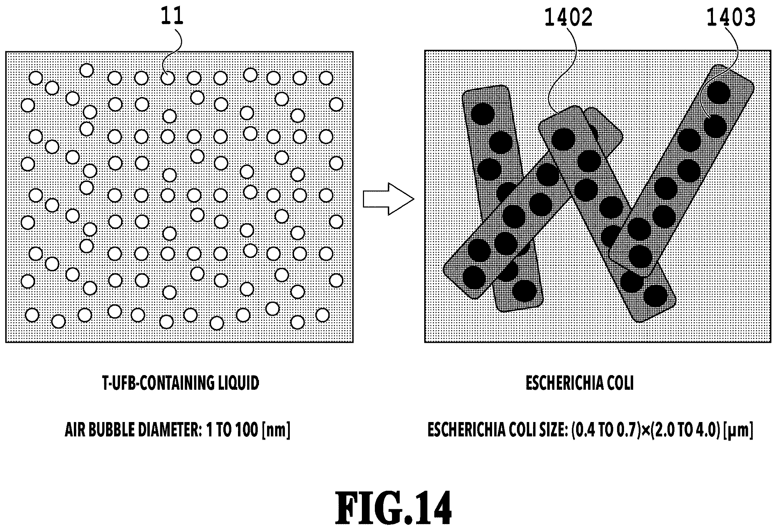

Fig. 14 is a diagram schematically illustrating a mechanism that Escherichia coli is killed by ozone UFBs. Escherichia coli has an intracellular structure 1403 in an outer wall 1402 in a long shape. On the other hand, the degerming liquid of the present example contains ozone UFBs 11 of 1 nm to 100 nm. It is considered that, out of those ozone UFBs 11, ones of especially small in size pass through the outer wall 1402, attack the intracellular structure 1403, and deactivate and dissolve the intracellular structure 1403.

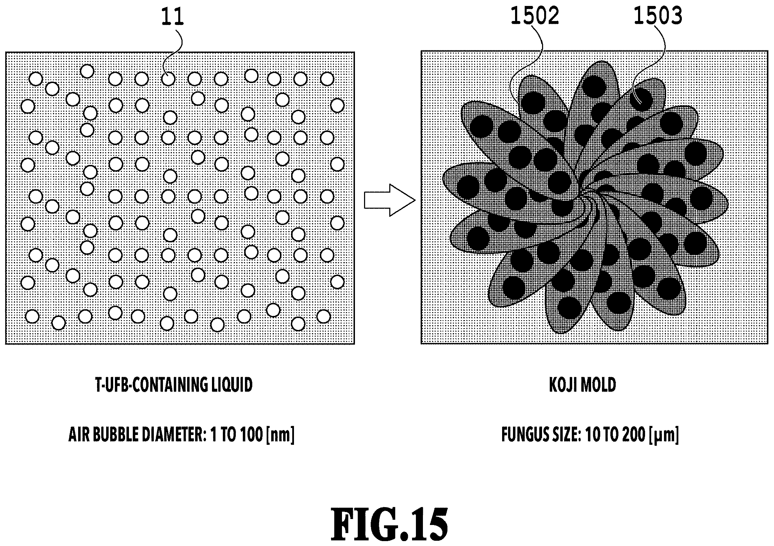

On the other hand, Fig. 15 is a diagram schematically illustrating a mechanism that koji mold is killed by the ozone UFBs 11. Koji mold also has an outer wall 1502 and a cell 1503 surrounded by the outer wall 1502. Also in such molds, it is considered that, ones of especially small in size out of the T-UFBs can pass through the outer wall 1502, and thus the cell 1503 is deactivated and dissolved by the offensive power of the ozone.

Additionally, the present inventors also conducted a following test in order to further confirm the effect of the degerming liquid of the present example.

First, thiosulfate was added as a reductant into common sample water, a reaction stop time was confirmed, and an oxidizing substance (residual chlorine or the like) was removed. Next, 0.1 mL of a fungus body suspension of each bacterium or mold was mixed with 10 mL of the above-described sample water, and the solution was adjusted and left such that a concentration of fungi in each solution is 105 to 106 pieces/mL. After the elapse of 72 hours, the solution was poured in agar plate and cultured at a predetermined temperature, and the number of remaining living bacteria was measured. The measuring number was n = 31 per fungus. With this testing method, substantially similar results as that in Fig. 13 could be obtained.

Note that, although an ozone gas is used to degerm or disinfect in the above-described test, as long as it is a sterilizing gas capable of deactivating a fungus body of bacteria or molds, other sterilizing gases such as nitrogen, carbon dioxide, and ethylene oxide can be used.

That is, according to the present example, a liquid in which a sterilizing gas is dissolved into the liquid and T-UFBs are generated by the T-UFB generating method can be effectively utilized as a degerming liquid that degerms bacteria and molds. Additionally, the degerming effect can be enhanced by applying predetermined ultrasonic wave vibration immediately before using as a degerming liquid.

### (Example 2)

In the present example, here is described an example of utilizing the T-UFB-containing liquid of the present embodiment as a liquid for purifying a liquid containing harmful heavy metal (hereinafter, referred to as a purifying liquid). The type of the gas to be contained in the T-UFBs in the purifying liquid of the present example is not particularly limited. As the gas to be contained in the T-UFBs, ozone may be used but also any gas can be used as long as it is a gas that can be dissolved into a liquid such as nitrogen, oxygen, carbon dioxide, hydrogen, or atmospheric air.

A specific testing method that the present inventors performed is described below. In the present test, as the harmful heavy metal, cyan, lead, hexavalent chromium, arsenic, and fluorine were prepared. Additionally, as the purifying liquid, three types of T-UFB-containing liquids in which the containing concentrations of the T-UFBs are different from each other were prepared.

First, the UFB generating apparatus 1 described in Fig. 1 is used to generate a UFB-containing liquid having the UFB concentration of 2.15×10e(+9) pieces/mL to be a first purifying liquid. Additionally, the first purifying liquid is diluted about 10-fold to be a second purifying liquid, and the first purifying liquid is diluted about 100-fold to be a third purifying liquid. For those first to third purifying liquids, an ultrasonic horn is inserted therein before being put in contact with the sample, and ultrasonic waves are oscillated for 30 seconds at 100 kHz and 800 W.

Next, each sample is added into the above-described purifying liquid stored in a volumetric flask of the capacity of 50 mL. Such addition is performed for all the combinations of the five types of samples and the first, second, and third purifying liquids. Then, each sample is adjusted to have a containing concentration in the solution of a value indicated below and is thereafter left for 72 hours.
- cyan: 1.0 mg/L (about 10 times higher than the environmental quality standards for soil) lead: 0.10 mg/L (same concentration as the environmental quality standards for soil)
- hexavalent chromium: 0.50 mg/L (amount 10 times higher than the environmental quality standards for soil)
- arsenic: 0.010 mg/L (same concentration as the environmental quality standards for soil)
- fluorine: 8.0 mg/L (amount 10 times higher than the environmental quality standards for soil)

After leaving for 72 hours, the concentration of each sample in the solution is measured. The measuring is performed in accordance with a method appropriate for each sample defined by the Japanese Industrial Standards (JIS). A measuring method appropriate for each sample is stated below.
- cyan : diphenylcarbazide absorptiometry
- lead: acid digestion-ICP mass spectrometry
- hexavalent chromium: diphenylcarbazide absorptiometry
- arsenic: acid digestion-ICP mass spectrometry
- fluorine: ion chromatographic method

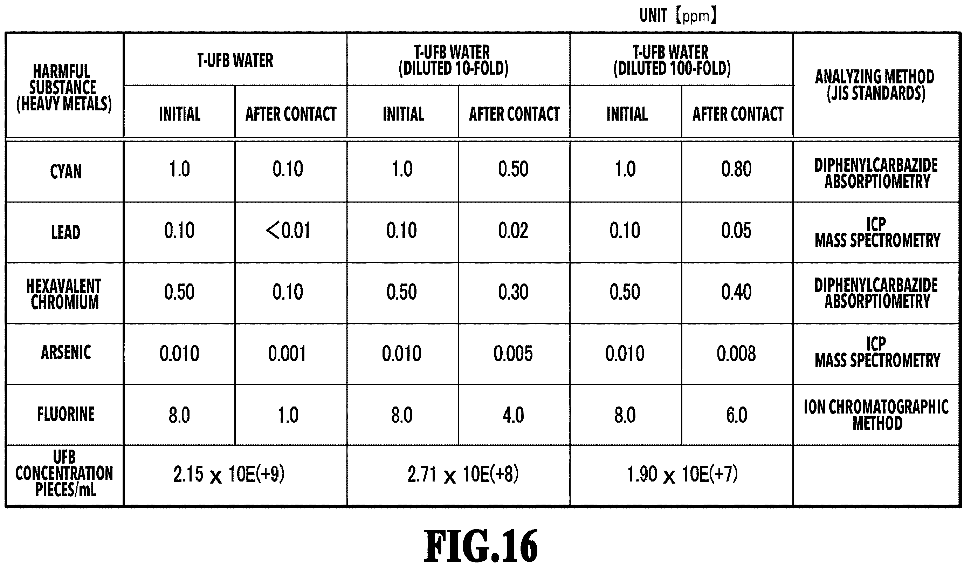

Fig. 16 illustrates a result of the above-described test. For each combination of the sample and the purifying liquid, the ion concentrations of the heavy metal contained in the solution are indicated while comparing between the initial stage of the contact and after the elapse of 72 hours. It can be seen in any of the combinations that the concentration of the harmful heavy metal is reduced and the solution is purified. Additionally, it can be seen that the purifying effect is higher as the purifying liquid with a greater T-UFB containing concentration.

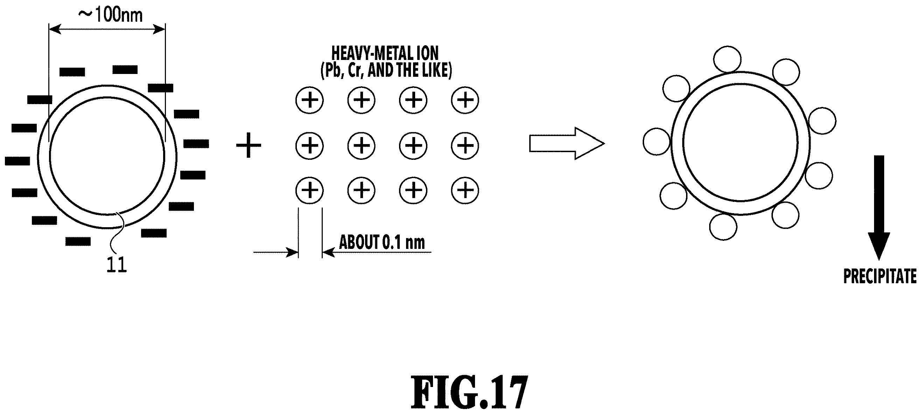

Fig. 17 is a schematic view illustrating a mechanism that a solution containing heavy metal is purified. In the purifying liquid of the present example, the contained UFBs 11 are hyperfine air bubbles of 100 nm or smaller, the surfaces are negatively charged at around -20 mV (±0 to -30 mV), and the UFBs 11 have Brownian motion in the liquid. On the other hand, in the solution, the heavy metal exists as a cation at +20 to +30 mV, and the size is around 0.1 nm.

Therefore, in the solution, the floating heavy-metal cation is in a state in which being attracted to the UFB 11 accompanying the surface of the UFB 11, and a phenomenon like a salting-out reaction occurs. Although the inside of the UFB is a gas, buoyancy acts hardly; for this reason, the bonding body in which multiple heavy-metal cations accompany such a UFB precipitates in the solution due to the gravity of the heavy metal. As a result, it is possible to remove the harmful heavy-metal ion in the solution, and the solution can be purified.

Note that, although a state in which multiple heavy-metal cations accompany one UFB 11 is illustrated in Fig. 17, a similar effect can be obtained with the UFB 11 in the form of the floc 11G as illustrated in Fig. 12. However, in order to precipitate more heavy-metal cations in a short time, it is preferable for the individual UFBs 11 to float individually. That is, by applying predetermined vibration to the purifying liquid in the present example before being put in contact with the sample, the dispersibility of the UFBs 11 can be enhanced, and the purifying effect of the purifying liquid can be further enhanced.

As described above, according to the present example, the UFB-containing liquid generated by the T-UFB generating method can be effectively utilized as a purifying liquid for purifying a solution containing harmful heavy metal.

### (Example 3)

In the present example, here is described an example of using the T-UFB-containing liquid of the present embodiment as a liquid for detoxifying a harmful organic substance (hereinafter, referred to as a detoxifying liquid).

In the present example, something in which an ozone gas is dissolved by the dissolving unit 200, and the T-UFBs are generated by the T-UFB generating unit 300 in the UFB generating apparatus 1 described in Fig. 1 is used as a detoxifying liquid.

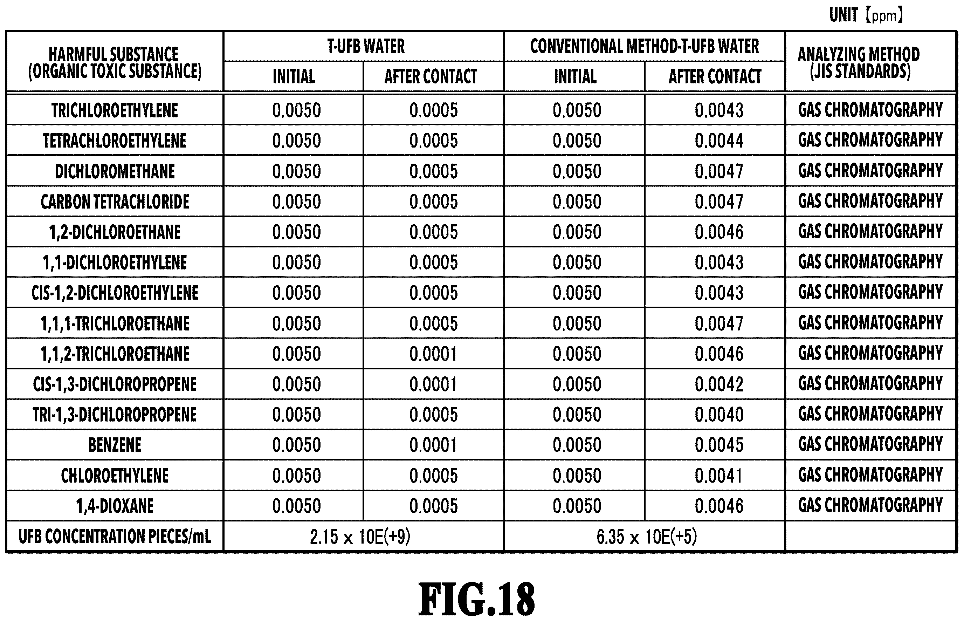

Fig. 18 is a diagram illustrating a detoxifying effect in the detoxifying liquid of the present example. In this case, the following 14 types of organic matters were prepared as the sample. They are trichloroethylene, tetrachloroethylene, dichloromethane, carbon tetrachloride, 1,2-dichloroethane, 1,1-dichloroethylene, cis-1,2-dichloroethylene, 1,1,1-trichloroethane, 1,1,2-trichloroethane, cis-1,3-dichloropropene, tri-1,3-dicchloropropene, benzene, chloroethylene, and 1,4-dioxane.

As the detoxifying liquid, in addition to the T-UFB-containing liquid of the present example, a UFB-containing liquid generated by a conventional method described in PTL 1 or PTL 2 was also prepared as a comparative example. While the UFB concentration of the detoxifying liquid of the present example was 2.15×10e(+9) pieces/mL, the UFB concentration of the comparative example was 6.35×10e(+5) pieces/mL.

As a testing method, each sample was added to each of the detoxifying liquid of the present example and the detoxifying liquid of the conventional method, and the concentration of each sample was adjusted to be 0.0050 ppm. Thereafter, the solution was left for 72 hours, and a concentration of each sample, that is, harmful substance in the mixed solution was measured. In this process, as the measuring method for the concentration, gas chromatography defined by the Japanese Industrial Standards (JIS) was employed.

According to Fig. 18, it can be seen in any of the samples that the containing concentration of the harmful substance is reduced by being put in contact with the ozone UFB-containing liquid. Additionally, it can be seen that the detoxifying effect of the UFB-containing liquid generated by the T-UFB generating method of the present example is higher than that of the UFB-containing liquid generated by the conventional method.

Ozone formed of three oxygen atoms is a gas that is colorless with unique irritating smell at normal temperature and has strong oxidizing power. Since ozone is stable as an oxygen gas after degradation, the concern for generation of a new harmful substance with the degradation is small. That is, an ozone gas has an excellent feature of reacting chemically with a persistent harmful organic matter, making it into low molecules, and changing it into a harmless organic matter. In a case where the T-UFB-containing liquid of the present example is used as the detoxifying liquid, many ozone UFBs in small size exist more than a case of using the conventional UFB-containing liquid; for this reason, it is estimated that the opportunity of a bonding group of the organic substance reacting with ozone is increased, and thus the detoxifying effect is enhanced. Additionally, with the ultrasonic waves applied to the detoxifying liquid, the frequency of the bonding group of the organic substance reacting with ozone is further increased, and thus the detoxifying effect can be further improved.

As described above, according to the present example, the ozone UFB-containing liquid generated by the T-UFB generating method can be effectively utilized as a detoxifying liquid for detoxifying a harmful organic substance.

### (Example 4)

In the present example, here is described an example of using the T-UFB-containing liquid of the present embodiment as a cleaning liquid for removing contamination in a glass tube. In the cleaning liquid of the present example, the type of the gas to be contained in the T-UFBs is not particularly limited. As the gas to be contained in the T-UFBs, ozone may be used, and as long as it is a gas that can be dissolved into a liquid such as nitrogen, oxygen, carbon dioxide, hydrogen, or atmospheric air, any liquid can be used.

A specific testing method that the present inventors performed is described below. In the present test, as a contamination component that is an evaluation target, the following three types of water solutions were prepared.
- 2.5% w/v casein sodium water solution 10 mL
- 0.5% w/v tannic acid water solution 10 mL
- 0.1% w/v methylene blue water solution 1 mL

Additionally, as the cleaning liquid, in addition to the T-UFB-containing liquid of the present example, the UFB-containing liquid created by the conventional method and tap water were also prepared as comparative examples. Note that, in the cleaning liquid of the present example, an ultrasonic horn is inserted, and ultrasonic waves were oscillated for 30 seconds at 150 kHz and 100 W immediately before the test.

As the testing method, first, the above-described three types of water solutions are mixed with each other in a beaker and agitated by a stirrer for 10 minutes or more to be an artificial contamination solution. Next, 20 µL of the artificial contamination solution is taken out from the beaker and applied to the inside of a glass tube. Then, the glass tube to which the artificial contamination solution was applied is dried for two hours in a constant temperature drying machine set at 115°C. Thereafter, the cleaning liquid was flowed in from one side of the glass tube, the purifying liquid was collected from the other side, and a TOC (Total Organic Carbon) amount contained in the collected purifying liquid was measured by a predetermined TOC measuring apparatus. Then, such measuring was performed on each of the three types of cleaning liquids, which are the T-UFB-containing liquid of the present embodiment, the UFB-containing liquid created by the conventional method, and tap water, while differing the flow-in time by stages.

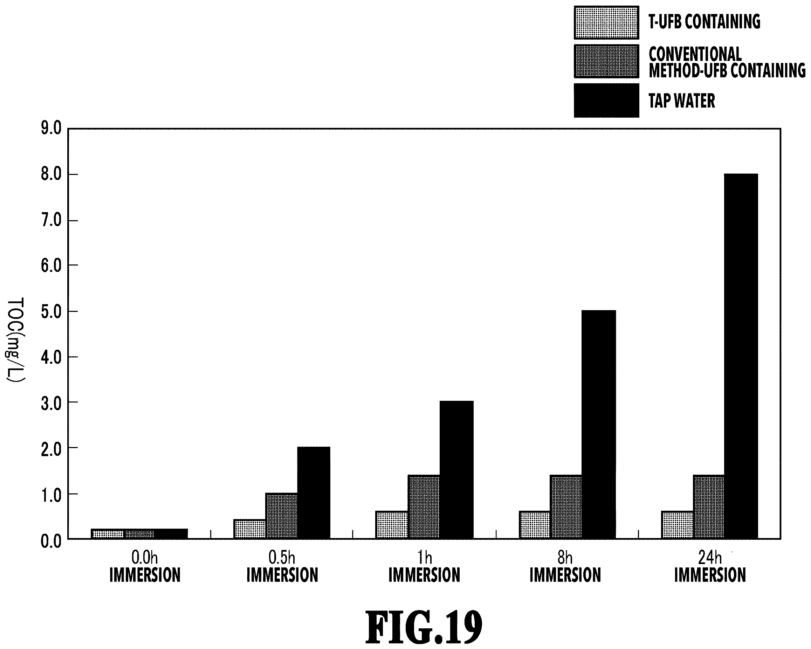

Fig. 19 is a diagram illustrating a result of the above-described test. In Fig. 19, vertical axis indicates the TOC amount, and as a value is greater, it means that the collection rate of contamination, that is, the cleaning effect of the cleaning liquid is higher. According to Fig. 19, although no difference occurs at the immersion into the cleaning liquid (0.0 h immersion), it can be seen that once the flowing of the cleaning liquid is started there occurs a difference in the cleaning effects depending on the cleaning liquid used. Specifically, the cleaning effect of the UFB-containing liquid is higher than that of tap water, and the cleaning effect of the T-UFB-containing liquid generated by the T-UFB generating method is further higher than that of the UFB-containing liquid generated by the conventional method. It is estimated that the mechanism of expressing the cleaning effect is that hyper small air bubbles of 100 nm or smaller are permeated an interface between a substrate (glass or the like) and the contamination (adhering matter), and thus interface debonding occurs (so-called liftoff effect).

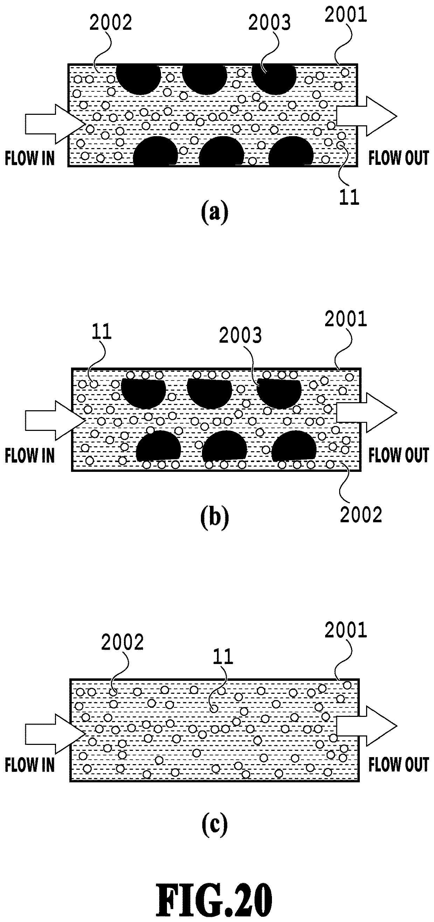

Figs. 20(a) to 20(c) are diagrams schematically illustrating states of removing contamination adhered to the glass tube by the cleaning liquid of the present example. There are illustrated states in which a cleaning liquid 2002 is flowed from left to right in Figs. 20(a) to 20(c) in a glass tube 2001 to which contamination 2003 is adhered. Fig. 20(a) illustrates immediately after the flow-in of the cleaning liquid is started. The contamination 2003 is adhered to the glass tube 2001, and many UFBs 11 are contained in the cleaning liquid 2002. Fig. 20(b) illustrates a state after the elapse of a predetermined time from the start of flowing. The UFBs 11 enter an interface between the glass tube 2001 and the contamination 2003 to lift the contamination 2003 off. Fig. 20(c) illustrates a state after the elapse of further time. The contamination 2003 lifted off by the UFBs 11 is flowed out with the cleaning liquid 2002, and the glass tube 2001 is cleaned thoroughly.

The cleaning liquid of the present example has smaller size of UFBs and a higher UFB concentration than that of the UFB-containing liquid generated by the conventional method. For this reason, it is considered in a case of using the cleaning liquid of the present example that the number of the UFBs 11 that enter the interface between the glass tube 2001 and the contamination 2003 is greater and thus the contamination is easily lifted off more than that in a case of using the UFB-containing liquid generated by the conventional method. Accordingly, it is expected that the cleaning liquid of the present example has a higher contamination liftoff effect than that of the UFB-containing liquid created by the conventional method and tap water, and thus the result illustrated in Fig. 19 is obtained.

Additionally, for example, if a sterilizing gas such as ozone is used as the gas to be contained in the T-UFBs, the contamination lifted off from the glass tube 2001 can be further dissolved or deactivated. Moreover, if ultrasonic waves are applied to the cleaning liquid before use, the substantive number and the dispersibility of the UFBs can be increased, and the contamination liftoff effect, that is, the cleaning effect can be further improved.

Note that, although the detail described above is that the contamination stuck to the inside of the glass tube 2001 is removed by flowing the UFB-containing liquid in the glass tube 2001, such a cleaning effect can be obtained not only in the glass tube 2001 as a matter of course. As long as the generated UFB-containing liquid can be flowed while being put in contact with any object, a contamination matter stuck to the object can be lifted off from the surface of the object to be cleaned out.

As described above, according to the present example, the UFB-containing liquid generated by the T-UFB generating method can be effectively utilized as a cleaning liquid for cleaning an object.

### [Second Embodiment]

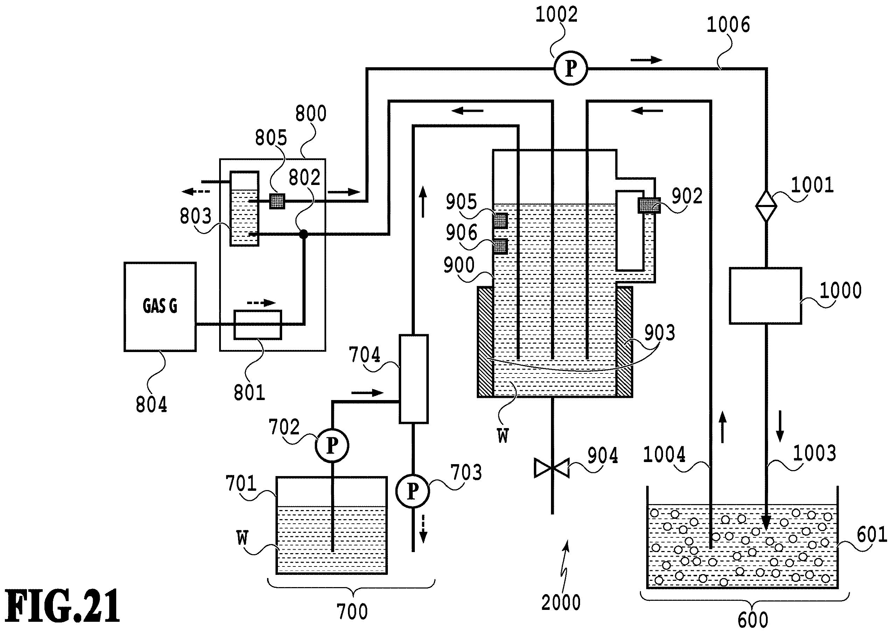

Fig. 21 is a schematic configuration view of a utilizing device 2000 for a UFB-containing liquid used in the present embodiment. The utilizing device 2000 of the present embodiment mainly includes a liquid supplying unit 700, a gas dissolving unit 800, a storing chamber 900, an ultra-fine bubble generating unit (UFB generating unit) 1000, and a purifying unit 600. The liquid supplying unit 700, the gas dissolving unit 800, and the UFB generating unit 1000 correspond to the pre-processing unit 100, the dissolving unit 200, and the T-UFB generating unit 300 in Fig. 1 described in the first embodiment, respectively. The units are connected by a piping 1006 with each other, and the liquid W is circulated by a pump 1002 arranged in the middle of the piping 1006. In Fig. 21, a solid line arrow indicates a flow of the liquid while a broken line arrow indicates a flow of the gas.

The liquid supplying unit 700 includes a liquid retaining unit 701, pumps 702 and 703, and a degassing unit 704. The liquid W retained in the liquid retaining unit 701 is transferred to the storing chamber 900 by the pumps 702 and 703 by way of the degassing unit 704. In the inside of the degassing unit 704, a film that allows the gas to pass therethrough but not the liquid is arranged. With only the gas passing through the film by pressures of the pumps 702 and 703, the gas and the liquid are separated from each other, and the liquid W moves toward the storing chamber 900 while the gas is ejected to the outside.

The gas dissolving unit 800 includes a gas supplying unit 804, a pre-processing unit 801, a converging unit 802, and a gas-liquid separating chamber 803. The gas supplying unit 804 may be a cylinder storing a predetermined gas G or may be an apparatus capable of continuously generating the predetermined gas G.

After performing processing such as discharging by the pre-processing unit 801, the gas G supplied by the gas supplying unit 804 converges in the converging unit 802 with the liquid W flowed out from the storing chamber 900. In this process, a part of the gas G is dissolved into the liquid W. The converged gas G and the liquid W are separated again by the gas-liquid separating chamber 803, and only the gas G that is not dissolved into the liquid W is ejected to the outside. The liquid W in which the gas G is dissolved is transferred to the UFB generating unit 1000 by the pump 1002 thereafter. A solubility sensor 805 for detecting the solubility of the gas G in the liquid W is provided downstream of the gas-liquid separating chamber 803.

The UFB generating unit 1000 generates UFBs in the liquid W flowed therein. The T-UFB generating method described using Figs. 4 to 10 is employed as the generating method for the UFBs. A filter 1001 is arranged upstream of the UFB generating unit 1000 to prevent impurities and dust from flowing into the UFB generating unit 1000. With impurities and dust removed, the UFB generating efficiency in the UFB generating unit 1000 can be improved. The UFB-containing liquid W generated in the UFB generating unit 1000 is stored in the storing chamber 900 through the piping 1006.

The storing chamber 900 stores a mixed liquid of the liquid W supplied from the liquid supplying unit 700, the liquid W in which the desired gas G is dissolved by the gas dissolving unit 800, and the UFB-containing liquid in which the T-UFBs are generated by the UFB generating unit 1000.

A temperature sensor 905 detects the temperature of the liquid stored in the storing chamber 900. A liquid surface sensor 902 is arranged at a predetermined height of the storing chamber 900 and detects a liquid surface of the liquid W in the storing chamber 900. A UFB concentration sensor 906 detects the UFB concentration of the liquid W stored in the storing chamber 900.

A temperature adjusting unit 903 manages the temperature of the liquid W stored in the storing chamber 900. In the dissolution of a desired gas G by the gas dissolving unit 800, it is more efficient with the temperature of the liquid W supplied to the gas dissolving unit 800 set as low as possible.

Although it is not illustrated in Fig. 21, an ultrasonic horn is arranged inside the storing chamber 900, and vibration is appropriately applied to the stored liquid. Additionally, an agitating means to uniform the temperature of the liquid W and the distribution of the UFBs may be provided in the storing chamber 900.

The purifying unit 600 mainly includes a purifying container 601, an inlet tube 1003, and an outlet tube 1004. In the purifying container 601, the liquid as a target of the purifying processing that contains harmful heavy metal is stored. The UBB containing liquid in which new UFBs are generated by the UFB generating unit 1000 is continuously supplied to the purifying container 601 through the inlet tube 1003. On the other hand, as for the liquid stored in the purifying container 601, a supernatant portion thereof (portion containing no precipitate) is continuously flowed out through the outlet tube 1004 and collected into the storing chamber 900.

In the utilizing device 2000 of the present embodiment, a member that is put in contact with the liquid W as the purifying liquid such as wetted portions of the piping 1006, the pump 1002, the filter 1001, the storing chamber 900, and the UFB generating unit 1000 is preferably formed of a material with strong resistance to corrosion. For example, fluorine system resin such as polytetrafluoroethylene (PTFE) and perfluoroalkoxy alkane (PFA), metal such as SUS316L, and other inorganic materials are able to be used favorably.

In the purifying container 601, the phenomenon described using Fig. 17 occurs between the floating heavy-metal cation and the UFB. That is, the floating heavy-metal cation is attracted to the UFB, accompanies the surface of the UFB, and precipitates with the UFB 1. In this process, although the UFB 11 is also consumed along with the precipitation of the heavy-metal cation, in the utilizing device 2000 of the present embodiment, the new UFBs 11 generated by the UFB generating unit 1000 are continuously supplied through the inlet tube 1003. Additionally, in the storing chamber 900, as described above, the floc including multiple UFBs is dispersed into multiple UFBs by applying vibration appropriately to the stored liquid. Therefore, in the purifying container 601, many UFBs with high dispersibility are supplied in sequence, and the remaining heavy-metal cations precipitate in sequence. Then, after the liquid is circulated for a predetermined period of time, the purified solution can be obtained by removing the precipitate in the container or collecting the supernatant liquid in the container.

Note that, the purifying unit 600 described in the present embodiment can also be changed to a unit with another function that is capable of using the UFB-containing liquid. For example, in a case where the gas G to be dissolved by the gas dissolving unit 800 is a sterilizing gas, and the liquid stored in the purifying container 601 is a solution containing bacteria and mold, the utilizing device 2000 illustrated in Fig. 21 can be a degerming or sterilizing device. In this case, with the many UFBs with high dispersibility continuously supplied into the container 600, the bacteria and mold remaining in the container 600 are deactivated and dissolved in sequence.

Additionally, in a case where the gas G to be dissolved by the gas dissolving unit 800 is an ozone gas, and the liquid stored in the container 600 is a solution containing harmful organic matters, the utilizing device 2000 illustrated in Fig. 21 can be a detoxifying device for the harmful organic matters. In this case, with the many ozone UFBs 11 with high dispersibility continuously supplied into the container 600, the harmful organic matters remaining in the container are made into low molecules and detoxified in sequence.

Moreover, with a contaminated objected stored in the container 600 in which the ultra-fine bubble-containing liquid flows, the utilizing device illustrated in Fig. 21 can be a cleaning device for the object. In this case, with the many UFBs with high dispersibility continuously supplied into the container 600, the contaminated matter stuck to the object can be lifted off from the surface of the object and can be floated and precipitated. In this process, if the gas G to be dissolved by the gas dissolving unit 800 is a sterilizing gas such as an ozone gas, the lifted contamination matter can be detoxified and deactivated.

As described above, according to the present embodiment, a highly concentrated UFB can be effectively utilized by the utilizing device 2000 of the present embodiment with the liquid circulated between the gas dissolving unit 800, the UFB generating unit 1000, and the purifying unit 600 (or utilizing unit).

The present application claims priority on the basis of Japanese Patent Application No. 2019-199138, filed on October 31, 2019 and Japanese Patent Application No. 2020-178854, filed on October 26, 2020, and the entirety of the described contents are incorporated herein.

### Reference Signs List

10 heating element
11 ultra-fine bubble (UFB)
11G floc

## Claims

1. A method for producing an ultra-fine bubble-containing liquid comprising:
an ultra-fine bubble generating step to generate an ultra-fine bubble in a liquid by heating a heating element and making film boiling on an interface between the liquid and the heating element; and
a dispersing step to disperse a floc, which includes two or more ultra-fine bubbles, into a plurality of ultra-fine bubbles by applying vibration to the liquid in which the floc floats.

2. The method for producing an ultra-fine bubble-containing liquid according to claim 1, further comprising:
a step of dissolving a predetermined gas into the liquid before the ultra-fine bubble generating step.

3. The method for producing an ultra-fine bubble-containing liquid according to claim 2, wherein
the predetermined gas includes at least one of oxygen, ozone, nitrogen, carbon dioxide, ethylene oxide, and atmospheric air.

4. An ultra-fine bubble-containing liquid, wherein
a floc, which includes a plurality of ultra-fine bubbles generated by heating a heating element and making film boiling on an interface between a liquid and the heating element, floats in the liquid.

5. The ultra-fine bubble-containing liquid according to claim 4, wherein
the ultra-fine bubbles are 100 nm or smaller in diameter and have a zeta potential of ±0 to -30 mV.

6. The ultra-fine bubble-containing liquid according to claim 5, wherein
the floc has a zeta potential of -60 to -100 mV.

7. The ultra-fine bubble-containing liquid according to any one of claims 4 to 6, wherein
the floc has a diameter of 100 to 200 nm.

8. The ultra-fine bubble-containing liquid according to any one of claims 4 to 7, wherein
the ultra-fine bubbles contain at least one of oxygen, ozone, nitrogen, carbon dioxide, ethylene oxide, and atmospheric air.

9. A method for utilizing ultra-fine bubbles comprising:
a dissolving step to dissolve a sterilizing gas into a liquid;
an ultra-fine bubble generating step to generate an ultra-fine bubble in the liquid by heating a heating element and making film boiling on an interface between the liquid and the heating element;
a dispersing step to disperse a floc, which includes two or more ultra-fine bubbles, into a plurality of ultra-fine bubbles by applying vibration to the liquid in which the floc floats; and
a degerming step to deactivate a bacterium or mold by putting an ultra-fine bubble-containing liquid obtained by the dispersing step into contact with the bacterium or mold.

10. The method for utilizing ultra-fine bubbles according to claim 9, wherein
the sterilizing gas includes a gas of at least one of ozone, nitrogen, carbon dioxide, and ethylene oxide.

11. A method for utilizing ultra-fine bubbles comprising:
an ultra-fine bubble generating step to generate an ultra-fine bubble in a liquid by heating a heating element and making film boiling on an interface between the liquid and the heating element;
a dispersing step to disperse a floc, which includes two or more ultra-fine bubbles, into a plurality of ultra-fine bubbles by applying vibration to the liquid in which the floc floats; and
a purifying step to detoxify a containing liquid of harmful heavy metal by putting the containing liquid into contact with an ultra-fine bubble-containing liquid obtained by the dispersing step.

12. The method for utilizing ultra-fine bubbles according to claim 11, wherein
the ultra-fine bubbles have a zeta potential of ±0 to -30 mV, and the harmful heavy metal is a cation in the containing liquid of the harmful heavy metal.

13. The method for utilizing ultra-fine bubbles according to claim 11 or 12, wherein
the harmful heavy metal is at least one of cyan, lead, hexavalent chromium, arsenic, and fluorine.

14. A method for utilizing ultra-fine bubbles comprising:
a dissolving step to dissolve an ozone gas into a liquid;
an ultra-fine bubble generating step to generate an ultra-fine bubble in the liquid by heating a heating element and making film boiling on an interface between the liquid and the heating element;
a dispersing step to disperse a floc, which includes two or more ultra-fine bubbles, into a plurality of ultra-fine bubbles by applying vibration to the liquid in which the floc floats; and
a step to detoxify a harmful organic matter by action of the ozone gas by putting an ultra-fine bubble-containing liquid obtained by the dispersing step into contact with the harmful organic matter.

15. The method for utilizing ultra-fine bubbles according to claim 14, wherein
the harmful organic matter is at least one of trichloroethylene, tetrachloroethylene, dichloromethane, carbon tetrachloride, 1,2-dichloroethane, 1,1-dichloroethylene, cis-1,2-dichloroethylene, 1,1,1-trichloroethane, 1,1,2-trichloroethane, cis-1,3-dichloroprepene, tri-1,3-dichloropropene, benzene, chloroethylene, and 1,4-dioxane.

16. A method for utilizing ultra-fine bubbles comprising:
an ultra-fine bubble generating step to generate an ultra-fine bubble in a liquid by heating a heating element and making film boiling on an interface between the liquid and the heating element;
a dispersing step to disperse a floc, which includes two or more ultra-fine bubbles, into a plurality of ultra-fine bubbles by applying vibration to the liquid in which the floc floats; and
a cleaning step to clean an object by flowing an ultra-fine bubble-containing liquid obtained by the dispersing step while putting into contact with the object.

17. The method for utilizing ultra-fine bubbles according to claim 16, wherein
the object is a tube, and in the cleaning step, the ultra-fine bubble-containing liquid flows inside the tube.

18. The method for utilizing ultra-fine bubbles according to any one of claims 9 to 17, wherein
the floc has a diameter of 100 to 200 nm in a stable state.

19. A device for utilizing ultra-fine bubbles comprising:
an ultra-fine bubble generating means to generate an ultra-fine bubble in a liquid by heating a heating element and making film boiling on an interface between the liquid and the heating element;
a dispersing means to disperse a floe, which includes two or more ultra-fine bubbles, into a plurality of ultra-fine bubbles by applying vibration to the liquid in which the floc floats;
a processing means to perform predetermined processing by using an ultra-fine bubble-containing liquid including the ultra-fine bubbles; and
a supplying means to supply a liquid from the ultra-fine bubble generating means to the processing means.

20. The device for utilizing ultra-fine bubbles according to claim 19, wherein
the processing means deactivates a bacterium or mold by putting the ultra-fine bubble-containing liquid into contact with the bacterium or mold.

21. The device for utilizing ultra-fine bubbles according to claim 19, wherein
the processing means detoxifies a containing liquid of harmful heavy metal by putting the containing liquid into contact with the ultra-fine bubble-containing liquid.

22. The device for utilizing ultra-fine bubbles according to claim 19, wherein
the processing means detoxifies a harmful organic matter by putting the harmful organic matter into contact with the ultra-fine bubble-containing liquid.

23. The device for utilizing ultra-fine bubbles according to claim 19, wherein
the processing means cleans an object by flowing the ultra-fine bubble-containing liquid while putting into contact with the object.

24. The device for utilizing ultra-fine bubbles according to any one of claims 19 to 23, wherein
the vibration is applied to the ultra-fine bubble-containing liquid by the dispersing means before the processing means performs the predetermined processing.

25. The device for utilizing ultra-fine bubbles according to any one of claims 19 to 24, further comprising:
a means to dissolve a predetermined gas into a liquid before the ultra-fine bubble generating means generates the ultra-fine bubbles.
